# EUROPEAN PATENT APPLICATION

(11) **EP 3 385 381 A1**
(43) Date of publication of application: **10.10.2018**
(21) Application number: 17165058.3
(22) Date of filing: 05.04.2017
(51) Int. Cl.: C12N 15/113

(54) **PRODUCTS AND COMPOSITIONS**

(71) Applicant: Silence Therapeutics GmbH, 13125 Berlin (DE)
(72) Inventor: The designation of the inventor has not yet been filed
(74) Representative: Stephen, Robert John

(57) **Abstract**

The present invention relates to products and compositions and their uses. In particular the invention relates to nucleic acid products that interfere with target gene expression or inhibit target gene expression and therapeutic uses of such products.

## Description

The present invention relates to products and compositions and their uses. In particular the invention relates to nucleic acid products that interfere with target gene expression or inhibit target gene expression and therapeutic uses of such products.

### Background

Double-stranded RNA (dsRNA) has been shown to block gene expression (Fire *et al,* 1998 and Elbashir *et al,* 2001) and this has been termed RNA interference (RNAi). Short dsRNAs direct gene-specific, post-transcriptional silencing in many organisms, including vertebrates, and has provided a new tool for studying gene function. RNAi is mediated by RNA-induced silencing complex (RISC), a sequence-specific, multi-component nuclease that destroys messenger RNAs homologous to the silencing trigger. Interfering RNA (iRNA) such as nucleic acid, antisense RNA, and micro-RNA are oligonucleotides that prevent the formation of proteins by gene-silencing i.e. inhibiting gene translation of the protein. Gene-silencing agents are becoming increasingly important for therapeutic applications in medicine.

However, delivery of nucleic acids, such as RNA, to cells avoiding degradation by cellular nucleases, whilst maintaining efficacy and target specificity has proved challenging to those in the field of developing nucleic acid molecules for therapeutic use.

According to Watts and Corey in the Journal of Pathology (2012; Vol 226, p 365-379) there are algorithms that can be used to design nucleic acids but none is perfect. It may take various experimental methods to identify potent nucleic acids, as algorithms do not take into account factors such as tertiary structure or the involvement of RNA binding proteins. Therefore the discovery of a potent nucleic acid with minimal off-target effects is a complex process but necessary for the pharmaceutical development of these highly charged molecules to be synthesised economically, distributed to target tissues, enter cells and function within acceptable limits of toxicity. Thus, means for efficient delivery of oligonucleotides, in particular double stranded siRNAs, to cells in vivo is becoming increasingly important and requires specific targeting and substantial protection from the extracellular environment, particularly serum proteins. One method of achieving specific targeting is to conjugate a targeting moiety to the iRNA duplex agent. The targeting moiety helps in targeting the iRNA duplex agent to the required target site and there is a need to design appropriate targeting moieties for the desired receptor sites for the conjugated molecules to be taken up by the cells such as by endocytosis.

However, targeting ligands developed so far do not always translate to in vivo settings and there is a clear need for more efficacious receptor specific ligand conjugated iRNA duplex agents and methods for their preparation for the in vivo delivery of oligonucleotide therapeutics, nucleic acids and double stranded siRNAs.

Rather than a lipid delivery system alone, the present invention addresses the structure of the nucleic acid itself. It has been unexpectedly found that a nucleic acid in accordance with the present invention has increased stability, which prevents degradation of the nucleic acid before entry into a cell.

### Summary of Invention

A first aspect of the invention relates to a nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of RNA transcribed from said target gene to be inhibited and wherein the terminal nucleotide at the 3' end of at least one of the first strand and the second strand is attached to the adjacent nucleotide via the 3' carbon of the terminal nucleotide and the 3' carbon of the adjacent nucleotide and/ or the terminal nucleotide at the 5' end of at least one of the first strand and the second strand is attached to the adjacent nucleotide via the 5' carbon of the terminal nucleotide and the 5' carbon of the adjacent nucleotide.

The 3' and/or 5' terminal nucleotides may be attached to the adjacent nucleotides via a phosphate group.

The first strand and second strand of the nucleic acid may be separate strands. The nucleic acid may comprise a single strand that comprises the first strand and the second strand.

The first strand and/or said second strand may be each from 17-35 nucleotides in length and the at least one duplex region may consist of 19-25 nucleotide base pairs.

The nucleic acid may: a) be blunt ended at both ends; b) have an overhang at one end and a blunt end at the other; or c) have an overhang at both ends.

One or more nucleotides on the first and/or second strand may be modified, to form modified nucleotides. One or more of the odd numbered nucleotides of the first strand may be modified. One or more of the even numbered nucleotides of the first strand may be modified by at least a second modification, wherein the at least second modification is different from the modification on the one or more odd nucleotides. At least one of the one or more modified even numbered nucleotides may be adjacent to at least one of the one or more modified odd numbered nucleotides.

A plurality of odd numbered nucleotides may be modified in the nucleic acid of the invention. A plurality of even numbered nucleotides may be modified by a second modification. The first strand may comprise adjacent nucleotides that are modified by a common modification. The first strand may also comprise adjacent nucleotides that are modified by a second different modification.

One or more of the odd numbered nucleotides of the second strand may be modified by a modification that is different to the modification on the odd nucleotides of the first strand and/or one or more of the even numbered nucleotides of the second strand may be modified by the same modification on the odd nucleotides of the first strand. At least one of the one or more modified even numbered nucleotides of the second strand may be adjacent to the one or more modified odd numbered nucleotides. A plurality of odd numbered nucleotides of the second strand may be modified by a common modification and/or a plurality of even numbered nucleotides may be modified by the same modification that is present on the first strand odd numbered nucleotides. A plurality of odd numbered nucleotides may be modified by a second modification, wherein the second modification is different to the modification on the first strand odd numbered nucleotides.

The second strand may comprise adjacent nucleotides that are modified by a common modification, which may be a second modification that is different from the modification on the odd numbered nucleotides of the first strand.

In the nucleic acid of the invention, each of the odd numbered nucleotides in the first strand and each of the even numbered nucleotides in the second strand may be modified with a common modification, and each of the even numbered nucleotides may be modified in the first strand with a second modification and each of the odd numbered nucleotides may be modified in the second strand with a second modification.

The nucleic acid of the invention may have the modified nucleotides of the first strand shifted by at least one nucleotide relative to the unmodified or differently modified nucleotides of the second strand.

The first strand may comprise a sequence selected form the group consisting of SEQ ID NO:s 1 to 5 and/or the second strand may comprise a sequence selected from the group consisting of SEQ ID NO:s 6 to 10.

The modification and/or modifications may each and individually be selected from the group consisting of 3'-terminal deoxy-thymine, 2'-O-methyl, a 2'-deoxy-modification, a 2'-amino-modification, a 2'-alkyl-modification, a morpholino modification, a phosphoramidate modification, 5'-phosphorothioate group modification, a 5' phosphate or 5' phosphate mimic modification and a cholesteryl derivative or a dodecanoic acid bisdecylamide group modification and/or the modified nucleotide may be any one of a locked nucleotide, an abasic nucleotide or a non-natural base comprising nucleotide. At least one modification may be 2'-O-methyl and/or at least one modification may be 2'-F.

In the nucleic acid of the invention, the terminal nucleotide at the 3' end of at least one of the first strand and the second strand that is attached to the adjacent nucleotide via the 3' carbon of the terminal nucleotide and the 3' carbon of the adjacent nucleotide and/ or the terminal nucleotide at the 5' end of at least one of the first strand and the second strand that is attached to the adjacent nucleotide via the 5' carbon of the terminal nucleotide and the 5' carbon of the adjacent nucleotide may be a purine.

The nucleic acid of the invention may further comprise a ligand.

A nucleic acid of the invention may comprise a phosphorothioate linkage between the terminal one, two or three 3' nucleotides and/or 5' nucleotides of the first and/or the second strand. It may comprise two phosphorothioate linkages between each of the three terminal 3' and between each of the three terminal 5' nucleotides on the first strand, and two phosphorothioate linkages between the three terminal nucleotides of the 3' end of the second strand.

The invention further provides, as a second aspect, a nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of a RNA transcribed from said target gene to be inhibited and wherein the terminal nucleotide at the 3' end of at least one of the first strand and the second strand is attached to the adjacent nucleotide via the 3' carbon of the terminal nucleotide and the 3' carbon of the adjacent nucleotide and/ or the terminal nucleotide at the 5' end of at least one of the first strand and the second strand is attached to the adjacent nucleotide via the 5' carbon of the terminal nucleotide and the 5' carbon of the adjacent nucleotide, and wherein the nucleic acid molecule is directly or indirectly conjugated to a ligand via a linker.

The ligand may comprise one or more GalNac ligands and derivatives thereof and the ligand may be directly or indirectly conjugated to a nucleic acid as defined in any preceding aspects by a bivalent or trivalent branched linker. The nucleotides may be modified as defined herein.

The ligand may comprise the formula I:

[S-X¹-P-X²]₃-A-X³-Z (I)

wherein:
S represents a saccharide, wherein the saccharide is N-acetyl galactosamine;
X¹ represents C₃-C₆ alkylene or (-CH₂-CH₂-O)ₘ(-CH₂)₂- wherein m is 1, 2, or 3;
P is a modified phosphate;
X² is alkylene or an alkylene ether of the formula (-CH₂)ₙO-CH₂- where n = 1- 6;
A is a branching unit;
X³ represents a bridging unit;
Z is the nucleic acid molecule;
and where the linkage between X³ and Z is a phosphate or thiophosphate.

The ligand may be any one of:

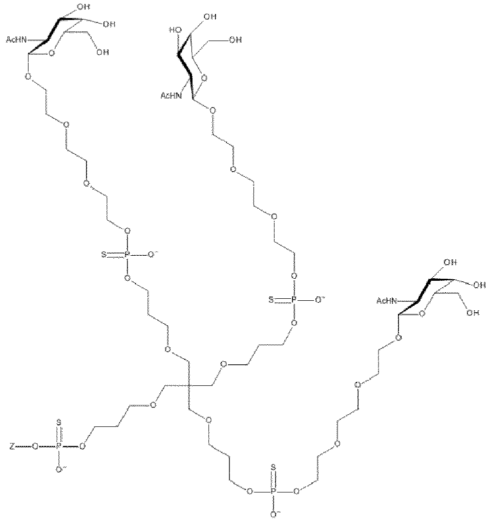

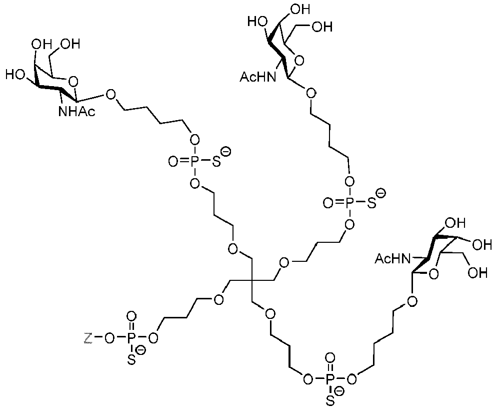

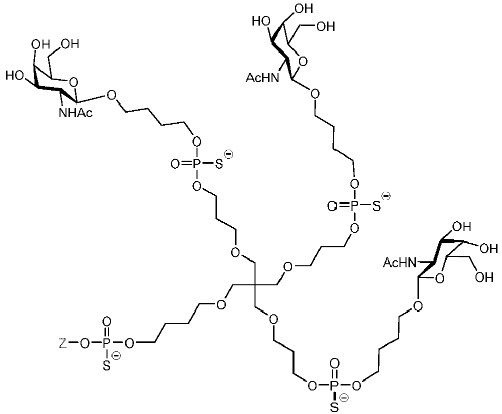

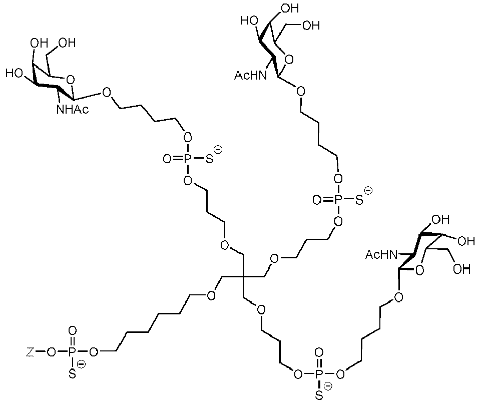

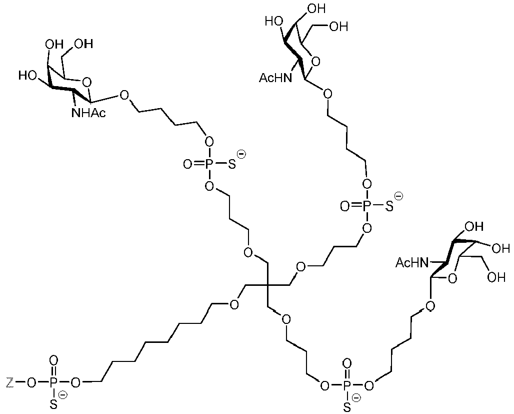

The ligand may comprise:

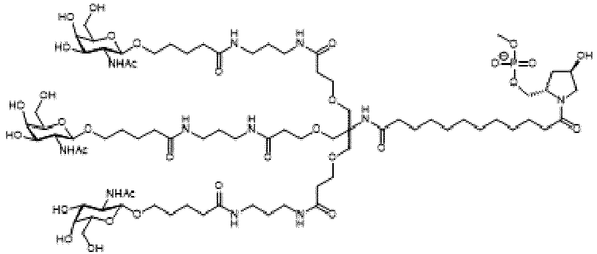

The invention also provides a composition comprising a nucleic acid as defined herein and a formulation comprising:
i) a cationic lipid, or a pharmaceutically acceptable salt thereof;
ii) a steroid;
iii) a phosphatidylethanolamine phospholipid;
iv) a PEGylated lipid.

The content of the cationic lipid component in the formulation may be from about 55 mol% to about 65 mol% of the overall lipid content of the lipid formulation, preferably about 59 mol% of the overall lipid content of the lipid formulation.

The composition may comprise a formulation comprising;
a cationic lipid having the structure; 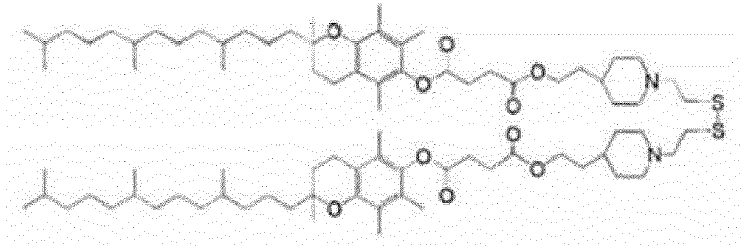
a steroid having the structure; 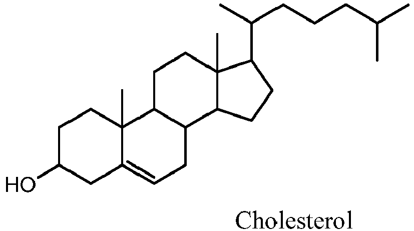
a phosphatidylethanolamine phospholipid having the structure; 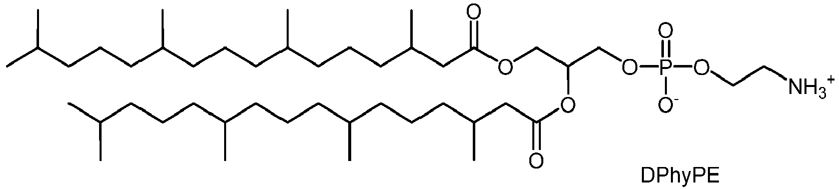
and a PEGylated lipid having the structure;

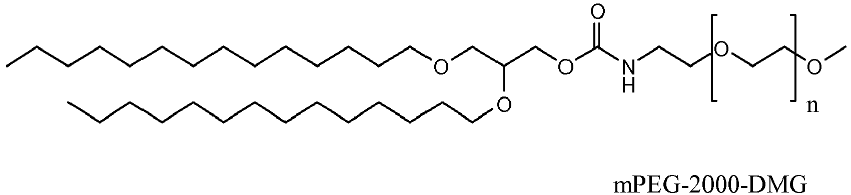

The invention also provides a composition comprising the nucleic acid of any aspect of the invention, and a physiologically acceptable excipient.

Also provided is a nucleic acid according to any aspect of the invention for use in the treatment of a disease or disorder and/or in the manufacture of a medicament for treating a disease or disorder.

The invention provides a method of treating a disease or disorder comprising administration of a composition comprising a nucleic acid according to any aspect of the invention to an individual in need of treatment. The nucleic acid may be administered to the subject subcutaneously or intravenously.

A method of making the nucleic acid according to the invention is also included.

### Detailed Description of Invention

The present invention relates to a nucleic acid which is double stranded and directed to an expressed RNA transcript of a target gene and compositions thereof. These nucleic acids can be used in the treatment of a variety of diseases and disorders where reduced expression of target gene products is desirable.

A first aspect of the invention relates to a nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of a RNA transcribed from said target gene to be inhibited and wherein the terminal nucleotide at the 3' end of at least one of the first strand and the second strand is attached to the adjacent nucleotide via the 3' carbon of the terminal nucleotide and the 3' carbon of the adjacent nucleotide and/ or the terminal nucleotide at the 5' end of at least one of the first strand and the second strand is attached to the adjacent nucleotide via the 5' carbon of the terminal nucleotide and the 5' carbon of the adjacent nucleotide.

By nucleic acid it is meant a nucleic acid comprising two strands comprising nucleotides, that is able to interfere with gene expression. Inhibition may be complete or partial and results in down regulation of gene expression in a targeted manner. The nucleic acid comprises two separate polynucleotide strands; the first strand, which may also be a guide strand; and a second strand, which may also be a passenger strand. The first strand and the second strand may be part of the same polynucleotide molecule that is self complementary which 'folds' to form a double stranded molecule. The nucleic acid may be an siRNA molecule.

The nucleic acid may comprise ribonucleotides, modified ribonucleotides, deoxynucleotides, deoxyribonucleotides, or nucleotide analogous. The nucleic acid may further comprise a double-stranded nucleic acid portion or duplex region formed by all or a portion of the first strand (also known in the art as a guide strand) and all or a portion of the second strand (also known in the art as a passenger strand). The duplex region is defined as beginning with the first base pair formed between the first strand and the second strand and ending with the last base pair formed between the first strand and the second strand, inclusive.

By duplex region it is meant the region in two complementary or substantially complementary oligonucleotides that form base pairs with one another, either by Watson-Crick base pairing or any other manner that allows for a duplex between oligonucleotide strands that are complementary or substantially complementary. For example, an oligonucleotide strand having 21 nucleotide units can base pair with another oligonucleotide of 21 nucleotide units, yet only 19 nucleotides on each strand are complementary or substantially complementary, such that the "duplex region" consists of 19 base pairs. The remaining base pairs may exist as 5' and 3' overhangs, or as single stranded regions. Further, within the duplex region, 100% complementarity is not required; substantial complementarity is allowable within a duplex region. Substantial complementarity refers to complementarity between the strands such that they are capable of annealing under biological conditions. Techniques to empirically determine if two strands are capable of annealing under biological conditions are well known in the art. Alternatively, two strands can be synthesised and added together under biological conditions to determine if they anneal to one another.

The portion of the first strand and second strand that form at least one duplex region may be fully complementary and are at least partially complementary to each other. Depending on the length of an nucleic acid, a perfect match in terms of base complementarity between the first strand and second strand is not necessarily required. However, the first and second strands must be able to hybridise under physiological conditions.

The complementarity between the first strand and second strand in the at least one duplex region may be perfect in that there are no nucleotide mismatches or additional/deleted nucleotides in either strand. Alternatively, the complementarity may not be perfect. The complementarity may be at least 70%, 75%, 80%, 85%, 90% or 95%.

The first strand and the second strand may each comprise a region of complementarity which comprises at least 15 contiguous nucleotides.

The nucleic acid involves the formation of a duplex region between all or a portion of the first strand and a portion of the target nucleic acid. The portion of the target nucleic acid that forms a duplex region with the first strand, defined as beginning with the first base pair formed between the first strand and the target sequence and ending with the last base pair formed between the first strand and the target sequence, inclusive, is the target nucleic acid sequence or simply, target sequence. The duplex region formed between the first strand and the second strand need not be the same as the duplex region formed between the first strand and the target sequence. That is, the second strand may have a sequence different from the target sequence however, the first strand must be able to form a duplex structure with both the second strand and the target sequence.

The complementarity between the first strand and the target sequence may be perfect (no nucleotide mismatches or additional/deleted nucleotides in either nucleic acid).

The complementarity between the first strand and the target sequence may not be perfect. The complementarity may be at least 70%, 80%, 85%, 90% or 95%.

The identity between the first strand and the complementary sequence of the target sequence may be at least 75%, 80%, 85%, 90% or 95%, provided a nucleic acid is capable of reducing or inhibiting the expression of a target gene.

The nucleic acid may be able to reduce expression of a target gene, by at least 25%, 50% or 75% of a comparative nucleic acid with perfect identity to the first strand and target sequence.

The nucleic acid may comprise a first strand and a second strand that are each from 19-25 nucleotides in length. The first strand and the second strand may be of different lengths.

The nucleic acid may be 15-25 nucleotide pairs in length. The nucleic acid may be 17-23 nucleotide pairs in length. The nucleic acid may be 17-25 nucleotide pairs in length. The nucleic acid may be 23-24 nucleotide pairs in length. The nucleic acid may be 19-21 nucleotide pairs in length. The nucleic acid may be 21-23 nucleotide pairs in length.

The nucleic acid may comprise a duplex region that consists of 19-25 nucleotide base pairs. The duplex region may consist of 17, 18, 19, 20, 21, 22, 23, 24 or 25 base pairs which may be contiguous.

The nucleic acid may be blunt ended at both ends; have an overhang at one end and a blunt end at the other end; or have an overhang at both ends.

An "overhang" as used herein has its normal and customary meaning in the art, i.e. a single stranded portion of a nucleic acid that extends beyond the terminal nucleotide of a complementary strand in a double strand nucleic acid. The term "blunt end" includes double stranded nucleic acid whereby both strands terminate at the same position, regardless of whether the terminal nucleotide(s) are base paired. The terminal nucleotide of a first strand and a second strand at a blunt end may be base paired. The terminal nucleotide of a first strand and a second strand at a blunt end may not be paired. The terminal two nucleotides of an first strand and a second strand at a blunt end may be base paired. The terminal two nucleotides of an first strand and a second strand at a blunt end may not be paired.

The nucleic acid may have an overhang at one end and a blunt end at the other. The nucleic acid may have an overhang at both ends. The nucleic acid may be blunt ended at both ends. The nucleic acid may be blunt ended at the end with the 5'-end of the first strand and the 3'-end of the second strand or at the 3'-end of the first strand and the 5'-end of the second strand.

The nucleic acid may comprise an overhang at a 3'- or 5'-end. The nucleic acid may have a 3'-overhang on the first strand. The nucleic acid may have a 3'-overhang on the second strand. The nucleic acid may have a 5'-overhang on the first strand. The nucleic acid may have a 5'-overhang on the second strand. The nucleic acid may have an overhang at both the 5'-end and 3'-end of the first strand. The nucleic acid may have an overhang at both the 5'-end and 3'-end of the second strand. The nucleic acid may have a 5' overhang on the first strand and a 3' overhang on the second strand. The nucleic acid may have a 3' overhang on the first strand and a 5' overhang on the second strand. The nucleic acid may have a 3' overhang on the first strand and a 3' overhang on the second strand. The nucleic acid may have a 5' overhang on the first strand and a 5' overhang on the second strand.

An overhang at the 3'-end or 5' end of the second strand or the first strand may be selected from consisting of 1, 2, 3, 4 and 5 nucleotides in length. Optionally, an overhang may consist of 1 or 2 nucleotides, which may or may not be modified.

Unmodified polynucleotides, particularly ribonucleotides, may be prone to degradation by cellular nucleases, and, as such, modification/modified nucleotides may be included in the nucleic acid of the invention.

One or more nucleotides on the second and/or first strand of the nucleic acid of the invention may be modified.

Modifications of the nucleic acid of the present invention generally provide a powerful tool in overcoming potential limitations including, but not limited to, in vitro and in vivo stability and bioavailability inherent to native RNA molecules. The nucleic acid according to the invention may be modified by chemical modifications. Modified nucleic acid can also minimise the possibility of inducing interferon activity in humans. Modification can further enhance the functional delivery of a nucleic acid to a target cell. The modified nucleic acid of the present invention may comprise one or more chemically modified ribonucleotides of either or both of the first strand or the second strand. A ribonucleotide may comprise a chemical modification of the base, sugar or phosphate moieties. The ribonucleic acid may be modified by substitution or insertion with analogues of nucleic acids or bases.

One or more nucleotides of a nucleic acid of the present invention may be modified. The nucleic acid may comprise at least one modified nucleotide. The modified nucleotide may be on the first strand. The modified nucleotide may be in the second strand. The modified nucleotide may be in the duplex region. The modified nucleotide may be outside the duplex region, i.e., in a single stranded region. The modified nucleotide may be on the first strand and may be outside the duplex region. The modified nucleotide may be on the second strand and may be outside the duplex region. The 3'-terminal nucleotide of the first strand may be a modified nucleotide. The 3'-terminal nucleotide of the second strand may be a modified nucleotide. The 5'-terminal nucleotide of the first strand may be a modified nucleotide. The 5'-terminal nucleotide of the second strand may be a modified nucleotide.

An nucleic acid of the invention may have 1 modified nucleotide or a nucleic acid of the invention may have about 2-4 modified nucleotides, or a nucleic acid may have about 4-6 modified nucleotides, about 6-8 modified nucleotides, about 8-10 modified nucleotides, about 10-12 modified nucleotides, about 12-14 modified nucleotides, about 14-16 modified nucleotides about 16-18 modified nucleotides, about 18-20 modified nucleotides, about 20-22 modified nucleotides, about 22-24 modified nucleotides, 24-26 modified nucleotides or about 26-28 modified nucleotides. In each case the nucleic acid comprising said modified nucleotides retains at least 50% of its activity as compared to the same nucleic acid but without said modified nucleotides. The nucleic acid may retain 55%, 60%, 65%, 70%, 75%, 80%, 85%, 90%, 95% or 100% or above of its activity as compared to the same nucleic acid but without said modified nucleotides

The modified nucleotide may be a purine or a pyrimidine. At least half of the purines may be modified. At least half of the pyrimidines may be modified. All of the purines may be modified. All of the pyrimidines may be modified. The modified nucleotides may be selected from the group consisting of a 3'-terminal deoxy-thymine (dT) nucleotide, a 2'-O-methyl modified nucleotide, a 2' modified nucleotide, a 2'-deoxy-modified nucleotide, a locked nucleotide, an abasic nucleotide, a 2'-amino-modified nucleotide, a 2'-alkyl-modified nucleotide, a morpholino nucleotide, a phosphoramidate, a non-natural base comprising nucleotide, a nucleotide comprising a 5'-phosphorothioate group, a nucleotide comprising a 5' phosphate or 5' phosphate mimic and a terminal nucleotide linked to a cholesteryl derivative or a dodecanoic acid bisdecylamide group.

The nucleic acid may comprise a nucleotide comprising a modified nucleotide, wherein the base is selected from 2-aminoadenosine, 2,6-diaminopurine,inosine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidine (e.g., 5-methylcytidine), 5-alkyluridine (e.g., ribothymidine), 5-halouridine (e.g., 5-bromouridine), 6-azapyrimidine, 6-alkylpyrimidine (e.g. 6-methyluridine), propyne, quesosine, 2-thiouridine, 4-thiouridine, wybutosine, wybutoxosine, 4-acetylcytidine, 5-(carboxyhydroxymethyl)uridine, 5'-carboxymethylaminomethyl-2-thiouridine, 5-carboxymethylaminomethyluridine, beta-D-galactosylqueosine, 1-methyladenosine, 1-methylinosine, 2,2-dimethylguanosine, 3-methylcytidine, 2-methyladenosine, 2-methylguanosine, N6-methyladenosine, 7-methylguanosine, 5-methoxyaminomethyl-2-thiouridine, 5-methylaminomethyluridine, 5-methylcarbonylmethyluridine, 5-methyloxyuridine, 5-methyl-2-thiouridine, 2-methylthio-N6-isopentenyladenosine, beta-D-mannosylqueosine, uridine-5-oxyacetic acid and 2-thiocytidine.

Nucleic acids discussed herein include unmodified RNA as well as RNA which have been modified, e.g., to improve efficacy, and polymers of nucleoside surrogates. Unmodified RNA refers to a molecule in which the components of the nucleic acid, namely sugars, bases, and phosphate moieties, are the same or essentially the same as that which occur in nature, for example as occur naturally in the human body. Modified nucleotide as used herein refers to a nucleotide in which one or more of the components of the nucleic acid, namely sugars, bases, and phosphate moieties, are different from that which occur in nature. While they are referred to as modified nucleotides they will of course, because of the modification, include molecules which are not nucleotides, for example a polynucleotide molecules in which the ribophosphate backbone is replaced with a non-ribophosphate construct that allows hybridisation between strands i.e. the modified nucleotides mimic the ribophosphate backbone.

Many of the modifications described below that occur within a nucleic acid will be repeated within a polynucleotide molecule, such as a modification of a base, or a phosphate moiety, or the a non-linking O of a phosphate moiety. In some cases the modification will occur at all of the possible positions/nucleotides in the polynucleotide but in many cases it will not. A modification may only occur at a 3' or 5' terminal position, may only occur in a terminal regions, such as at a position on a terminal nucleotide or in the last 2, 3, 4, 5, or 10 nucleotides of a strand. A modification may occur in a double strand region, a single strand region, or in both. A modification may occur only in the double strand region of an nucleic acid of the invention or may only occur in a single strand region of an nucleic acid of the invention. A phosphorothioate modification at a non-linking O position may only occur at one or both termini, may only occur in a terminal region, e.g., at a position on a terminal nucleotide or in the last 2, 3, 4 or 5 nucleotides of a strand, or may occur in duplex and/or in single strand regions, particularly at termini. The 5' end or 3' ends may be phosphorylated.

Stability of an nucleic acid of the invention may be increased by including particular bases in overhangs, or to include modified nucleotides, in single strand overhangs, e.g., in a 5'or 3' overhang, or in both. Purine nucleotides may be included in overhangs. All or some of the bases in a 3' or 5' overhang may be modified. Modifications can include the use of modifications at the 2' OH group of the ribose sugar, the use of deoxyribonucleotides, instead of ribonucleotides, and modifications in the phosphate group, such as phosphothioate modifications. Overhangs need not be homologous with the target sequence.

Nucleases can hydrolyze nucleic acid phosphodiester bonds. However, chemical modifications to nucleic acids can confer improved properties, and, can render oligoribonucleotides more stable to nucleases.

Modified nucleic acids, as used herein, can include one or more of:
(i) alteration, e.g., replacement, of one or both of the non-linking phosphate oxygens and/or of one or more of the linking phosphate oxygens (referred to as linking even if at the 5' and 3' terminus of the nucleic acid of the invention);
(ii) alteration, e.g., replacement, of a constituent of the ribose sugar, e.g., of the 2' hydroxyl on the ribose sugar;
(iii) replacement of the phosphate moiety with "dephospho" linkers;
(iv) modification or replacement of a naturally occurring base;
(v) replacement or modification of the ribose-phosphate backbone;
(vi) modification of the 3' end or 5' end of the RNA, e.g., removal, modification or replacement of a terminal phosphate group or conjugation of a moiety, e.g., a fluorescently labeled moiety, to either the 3' or 5' end of RNA.

The terms replacement, modification, alteration, indicates a difference from a naturally occurring molecule.

Specific modifications are discussed in more detail below.

Examples of modified phosphate groups include phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulphur. One, each or both non-linking oxygens in the phosphate group can be independently any one of S, Se, B, C, H, N, or OR (R is alkyl or aryl).

The phosphate linker can also be modified by replacement of a linking oxygen with nitrogen (bridged phosphoroamidates), sulfur (bridged phosphorothioates) and carbon (bridged methylenephosphonates). The replacement can occur at a terminal oxygen. Replacement of the non-linking oxygens with nitrogen is possible.

A modified nucleotide can include modification of the sugar groups. The 2' hydroxyl group (OH) can be modified or replaced with a number of different "oxy" or "deoxy" substituents.

Examples of "oxy"-2' hydroxyl group modifications include alkoxy or aryloxy (OR, e.g., R=H, alkyl, cycloalkyl, aryl, aralkyl, heteroaryl or sugar); polyethyleneglycols (PEG), O(CH2CH2O)nCH2CH2OR; "locked" nucleic acids (LNA) in which the 2' hydroxyl is connected, e.g., by a methylene bridge, to the 4' carbon of the same ribose sugar; O-AMINE (AMINE=NH2; alkylamino, dialkylamino, heterocyclyl, arylamino, diaryl amino, heteroaryl amino, or diheteroaryl amino, ethylene diamine, polyamino) and aminoalkoxy, O(CH2)nAMINE, (e.g., AMINE=NH2; alkylamino, dialkylamino, heterocyclyl, arylamino, diaryl amino, heteroaryl amino, or diheteroaryl amino, ethylene diamine, polyamino).

"Deoxy" modifications include hydrogen halo; amino (e.g., NH2; alkylamino, dialkylamino, heterocyclyl, arylamino, diaryl amino, heteroaryl amino, diheteroaryl amino, or amino acid); NH(CH2CH2NH)nCH2CH2-AMINE (AMINE=NH2; alkylamino, dialkylamino, heterocyclyl, arylamino, diaryl amino, heteroaryl amino, or diheteroaryl amino), -NHC(O)R (R=alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino functionality. Other substitutents of certain embodiments include 2'-methoxyethyl, 2'-OCH3, 2'-O-allyl, 2'-C-allyl, and 2'-fluoro.

The sugar group can also contain one or more carbons that possess the opposite stereochemical configuration than that of the corresponding carbon in ribose. Thus, a modified nucleotides may contain a sugar such as arabinose.

Modified nucleotides can also include "abasic" sugars, which lack a nucleobase at C-l'. These abasic sugars can further contain modifications at one or more of the constituent sugar atoms.

The 2' modifications may be used in combination with one or more phosphate linker modifications (e.g., phosphorothioate).

The phosphate group can be replaced by non-phosphorus containing connectors.

Examples of moieties which can replace the phosphate group include siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino. In certain embodiments, replacements may include the methylenecarbonylamino and methylenemethylimino groups.

The phosphate linker and ribose sugar may be replaced by nuclease resistant nucleotides.

Examples include the morpholino, cyclobutyl, pyrrolidine and peptide nucleic acid (PNA) nucleoside surrogates. In certain embodiments, PNA surrogates may be used.

The 3' and 5' ends of an oligonucleotide can be modified. Such modifications can be at the 3' end or the 5' end or both ends of the molecule. They can include modification or replacement of an entire terminal phosphate or of one or more of the atoms of the phosphate group. For example, the 3' and 5' ends of an oligonucleotide can be conjugated to other functional molecular entities such as labeling moieties, e.g., fluorophores (e.g., pyrene, TAMRA, fluorescein, Cy3 or Cy5 dyes) or protecting groups (based e.g., on sulfur, silicon, boron or ester). The functional molecular entities can be attached to the sugar through a phosphate group and/or a linker. The terminal atom of the linker can connect to or replace the linking atom of the phosphate group or the C-3' or C-5' O, N, S or C group of the sugar. Alternatively, the linker can connect to or replace the terminal atom of a nucleotide surrogate (e.g., PNAs). These spacers or linkers can include e.g., -(CH2)n-, -(CH2)nN-, -(CH2)nO-, -(CH2)nS-, O(CH2CH2O)nCH2CH2OH (e.g., n=3 or 6), abasic sugars, amide, carboxy, amine, oxyamine, oxyimine, thioether, disulfide, thiourea, sulfonamide, or morpholino, or biotin and fluorescein reagents. The 3' end can be an -OH group.

Other examples of terminal modifications include dyes, intercalating agents (e.g., acridines), cross-linkers (e.g., psoralene, mitomycin C), porphyrins (TPPC4, texaphyrin, Sapphyrin), polycyclic aromatic hydrocarbons (e.g., phenazine, dihydrophenazine), artificial endonucleases (e.g., EDTA), lipophilic carriers (e.g., cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g., biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles).

Terminal modifications can be added for a number of reasons, including to modulate activity or to modulate resistance to degradation. Terminal modifications useful for modulating activity include modification of the 5' end with phosphate or phosphate analogs. Nucleic acids of the invention, on the first or second strand, may be 5' phosphorylated or include a phosphoryl analog at the 5' prime terminus. 5'-phosphate modifications include those which are compatible with RISC mediated gene silencing. Suitable modifications include: 5'-monophosphate ((HO)2(O)P--O-5'); 5'-diphosphate ((HO)2(O)P--O--P(HO)(O)--O-5'); 5'-triphosphate ((HO)2(O)P--O--(HO)(O)P--O--P(HO)(O)--O-5'); 5'-guanosine cap (7-methylated or non-methylated) (7m-G-O-5-(HO)(O)P--O--(HO)(O)P--O--P(HO)(O)--O-5'); 5'-adenosine cap (Appp), and any modified or unmodified nucleotide cap structure (N-O-5'-(HO)(O)P-O-(HO)(O)P-O-P(HO)(O)--O-5'); 5'-monothiophosphate (phosphorothioate; (HO)2(S)P--O-5'); 5'-monodithiophosphate (phosphorodithioate; (HO)(HS)(S)P--O-5'), 5'-phosphorothiolate ((HO)2(O)P--S-5'); any additional combination of oxygen/sulfur replaced monophosphate, diphosphate and triphosphates (e.g., 5'-alpha-thiotriphosphate, 5'-gamma-thiotriphosphate, etc.), 5'-phosphoramidates ((HO)2(O)P-NH-5', (HO)(NH2)(O)P--O-5'), 5'-alkylphosphonates (R=alkyl=methyl, ethyl, isopropyl, propyl, etc., e.g., RP(OH)(O)-O-5'-, (OH)2(O)P-5-CH2-), 5'vinylphosphonate, 5'-alkyletherphosphonates (R=alkylether=methoxymethyl (MeOCH2-), ethoxymethyl, etc., e.g., RP(OH)(O)--O-5'-).

The nucleic acid of the present invention may include one or more phosphorothioate modifications on one or more of the terminal ends of the first and/or the second strand. Optionally, each or either end of the first strand may comprise one or two or three phosphorothioate modified nucleotides. Optionally, each or either end of the second strand may comprise one or two or three phosphorothioate modified nucleotides. Optionally, both ends of the first strand and the 5' end of the second strand may comprise two phosphorothioate modified nucleotides. By phosphorothioate modified nucleotide it is meant that the linkage between the nucleotide and the adjacent nucleotide comprises a phosphorothioate group instead of a standard phosphate group.

Terminal modifications can also be useful for monitoring distribution, and in such cases the groups to be added may include fluorophores, e.g., fluorscein or an Alexa dye. Terminal modifications can also be useful for enhancing uptake, useful modifications for this include cholesterol. Terminal modifications can also be useful for cross-linking an RNA agent to another moiety.

Adenine, guanine, cytosine and uracil are the most common bases found in RNA. These bases can be modified or replaced to provide RNA's having improved properties. E.g., nuclease resistant oligoribonucleotides can be prepared with these bases or with synthetic and natural nucleobases (e.g., inosine, thymine, xanthine, hypoxanthine, nubularine, isoguanisine, or tubercidine) and any one of the above modifications. Alternatively, substituted or modified analogs of any of the above bases and "universal bases" can be employed. Examples include 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine, 7-deazaadenine, N6,N6-dimethyladenine, 2,6-diaminopurine, 5-amino-allyl-uracil, N3-methyluracil, substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3-carboxypropyl)uracil, 3-methylcytosine, 5-methylcytosine, N<4>-acetyl cytosine, 2-thiocytosine, N6-methyladenine, N6-isopentyladenine, 2-methylthio-N6-isopentenyladenine, N-methylguanines, or O-alkylated bases.

As used herein, the terms "non-pairing nucleotide analog" means a nucleotide analog which includes a non-base pairing moiety including but not limited to: 6 des amino adenosine (Nebularine), 4-Me-indole, 3-nitropyrrole, 5-nitroindole, Ds, Pa, N3-Me ribo U, N3-Me riboT, N3-Me dC, N3-Me-dT, N1-Me-dG, N1-Me-dA, N3-ethyl-dC, N3-Me dC. In some embodiments the non-base pairing nucleotide analog is a ribonucleotide. In other embodiments it is a deoxyribonucleotide.

As used herein, the term, "terminal functional group" includes without limitation a halogen, alcohol, amine, carboxylic, ester, amide, aldehyde, ketone, ether groups.

Certain moieties may be linked to the 5' terminus of the first strand or the second strand and includes abasic ribose moiety, abasic deoxyribose moiety, modifications abasic ribose and abasic deoxyribose moieties including 2' O alkyl modifications; inverted abasic ribose and abasic deoxyribose moieties and modifications thereof, C6-imino-Pi; a mirror nucleotide including L-DNA and L-RNA; 5'OMe nucleotide; and nucleotide analogs including 4',5'-methylene nucleotide; 1-(β-D-erythrofuranosyl)nucleotide; 4'-thio nucleotide, carbocyclic nucleotide; 5'-amino-alkyl phosphate; 1,3-diamino-2-propyl phosphate, 3-aminopropyl phosphate; 6-aminohexyl phosphate; 12-aminododecyl phosphate; hydroxypropyl phosphate; 1,5-anhydrohexitol nucleotide; alpha-nucleotide; threo-pentofuranosyl nucleotide; acyclic 3',4'-seco nucleotide; 3,4-dihydroxybutyl nucleotide; 3,5-dihydroxypentyl nucleotide, 5'-5'-inverted abasic moiety; 1,4-butanediol phosphate; 5'-amino; and bridging or non bridging methylphosphonate and 5'-mercapto moieties. The nucleic acids of the invention may be included as one or more inverted nucleotides, for example inverted thymidine or inverted adenine (for example see Takei, et al., 2002. JBC 277 (26):23800-06).

As used herein, the term "inhibit", "down-regulate", or "reduce" with respect to gene expression means the expression of the gene, or level of RNA molecules or equivalent RNA molecules encoding one or more proteins or protein subunits (e.g., mRNA), or activity of one or more proteins or protein subunits, is reduced below that observed in the absence of a nucleic acid of the invention; for example the expression may be reduced to 90%, 80%, 70%, 60%, 50%, 40%, 30%, 20%, 10%, 5% or less than that observed in the absence of an inhibitor.

The nucleic acid of the present invention may comprise an abasic nucleotide. The term "abasic" as used herein, refers to moieties lacking a base or having other chemical groups in place of a base at the 1' position, for example a 3',3'-linked or 5',5'-linked deoxyabasic ribose derivative.

The nucleic acid may comprise one or more nucleotides on the second and/or first strands that are modified. Alternating nucleotides may be modified, to form modified nucleotides.

Alternating as described herein means to occur one after another in a regular way. In other words, alternating means to occur in turn repeatedly. For example if one nucleotide is modified, the next contiguous nucleotide is not modified and the following contiguous nucleotide is modified and so on. One nucleotide may be modified with a first modification, the next contiguous nucleotide may be modified with a second modification and the following contiguous nucleotide is modified with the first modification and so on, where the first and second modifications are different.

One or more of the odd numbered nucleotides of the first strand of the nucleic acid of the invention may be modified wherein the first strand is numbered 5' to 3'. The term "odd numbered" as described herein means a number not divisible by two. Examples of odd numbers are 1, 3, 5, 7, 9, 11 and so on. One or more of the even numbered nucleotides of the first strand of the nucleic acid of the invention may be modified, wherein the first strand is numbered 5' to 3'. The term "even numbered" as described herein means a number which is evenly divisible by two. Examples of even numbers are 2, 4, 6, 8, 10, 12, 14 and so on. One or more of the odd numbered nucleotides of the second strand of the nucleic acid of the invention may be modified wherein the second strand is numbered 3' to 5'. One or more of the even numbered nucleotides of the second strand of the nucleic acid of the invention may be modified, wherein the second strand is numbered 3' to 5'.

One or more nucleotides on the first and/or second strand may be modified, to form modified nucleotides. One or more of the odd numbered nucleotides of the first strand may be modified. One or more of the even numbered nucleotides of the first strand may be modified by at least a second modification, wherein the at least second modification is different from the modification on the one or more add nucleotides. At least one of the one or more modified even numbered nucleotides may be adjacent to at least one of the one or more modified odd numbered nucleotides.

A plurality of odd numbered nucleotides in the first strand may be modified in the nucleic acid of the invention. A plurality of even numbered nucleotides in the first strand may be modified by a second modification. The first strand may comprise adjacent nucleotides that are modified by a common modification. The first strand may also comprise adjacent nucleotides that are modified by a second different modification.

One or more of the odd numbered nucleotides of the second strand may be modified by a modification that is different to the modification of the odd numbered nucleotides on the first strand and/or one or more of the even numbered nucleotides of the second strand may be by the same modification of the odd numbered nucleotides of the first strand. At least one of the one or more modified even numbered nucleotides of the second strand may be adjacent to the one or more modified odd numbered nucleotides. A plurality of odd numbered nucleotides of the second strand may be modified by a common modification and/or a plurality of even numbered nucleotides may be modified by the same modification that is present on the first stand odd numbered nucleotides. A plurality of odd numbered nucleotides on the second strand may be modified by a second modification, wherein the second modification is different from the modification of the first strand odd numbered nucleotides.

The second strand may comprise adjacent nucleotides that are modified by a common modification, which may be a second modification that is different from the modification of the odd numbered nucleotides of the first strand.

In the nucleic acid of the invention, each of the odd numbered nucleotides in the first strand and each of the even numbered nucleotides in the second strand may be modified with a common modification and, each of the even numbered nucleotides may be modified in the first strand with a second modification and each of the odd numbered nucleotides may be modified in the second strand with the second modification.

The nucleic acid of the invention may have the modified nucleotides of the first strand shifted by at least one nucleotide relative to the unmodified or differently modified nucleotides of the second strand.

One or more or each of the odd numbered nucleotides may be modified in the first strand and one or more or each of the even numbered nucleotides may be modified in the second strand. One or more or each of the alternating nucleotides on either or both strands may be modified by a second modification. One or more or each of the even numbered nucleotides may be modified in the first strand and one or more or each of the even numbered nucleotides may be modified in the second strand. One or more or each of the alternating nucleotides on either or both strands may be modified by a second modification. One or more or each of the odd numbered nucleotides may be modified in the first strand and one or more of the odd numbered nucleotides may be modified in the second strand by a common modification. One or more or each of the alternating nucleotides on either or both strands may be modified by a second modification. One or more or each of the even numbered nucleotides may be modified in the first strand and one or more or each of the odd numbered nucleotides may be modified in the second strand by a common modification. One or more or each of the alternating nucleotides on either or both strands may be modified by a second modification.

The nucleic acid of the invention may comprise single or double stranded constructs that comprise at least two regions of alternating modifications in one or both of the strands. These alternating regions can comprise up to about 12 nucleotides but preferably comprise from about 3 to about 10 nucleotides. The regions of alternating nucleotides may be located at the termini of one or both strands of the nucleic acid of the invention. The nucleic acid may comprise from 4 to about 10 nucleotides of alternating nucleotides at each termini (3' and 5') and these regions may be separated by from about 5 to about 12 contiguous unmodified or differently or commonly modified nucleotides.

The odd numbered nucleotides of the first strand may be modified and the even numbered nucleotides may be modified with a second modification. The second strand may comprise adjacent nucleotides that are modified with a common modification, which may be the same as the modification of the odd numbered nucleotides of the first strand. One or more nucleotides of second strand may also be modified with the second modification. One or more nucleotides with the second modification may be adjacent to each other and to nucleotides having a modification that is the same as the modification of the odd numbered nucleotides of the first strand. The first strand may also comprise phosphorothioate linkages between the two nucleotides at the 3' end and at the 5' end. The second strand may comprise a phosphorothioate linkage between the two nucleotides at 5' end. The second strand may also be conjugated to a ligand at the 5' end.

The nucleic acid of the invention may comprise a first strand comprising adjacent nucleotides that are modified with a common modification. One or more of such nucleotides may be adjacent to one or more nucleotides which may be modified with a second modification. One or more nucleotides with the second modification may be adjacent. The second strand may comprise adjacent nucleotides that are modified with a common modification, which may be the same as one of the modifications of one or more nucleotides of the first strand. One or more nucleotides of second strand may also be modified with the second modification. One or more nucleotides with the second modification may be adjacent. The first strand may also comprise phosphorothioate linkages between the two nucleotides at the 5' end and at the 3' end. The second strand may comprise a phosphorothioate linkage between the two nucleotides at 3' end. The second strand may also be conjugated to a ligand at the 5' end.

The nucleotides numbered (from 5' to 3' on the first strand and 3' and 5' on the second strand) 1, 3, 5, 7, 9, 11, 13, 15, 17, 19, 21, 23 and 25 may be modified by a modification on the first strand. The nucleotides numbered 2, 4, 6, 8, 10, 12, 14, 16, 18, 20, 22 and 24 may be modified by a second modification on the first strand. The nucleotides numbered 1, 3, 5, 7, 9, 11, 13, 15, 17, 19, 21, 23 may be modified by a modification on the second strand. The nucleotides numbered 2, 4, 6, 8, 10, 12, 14, 16, 18, 20, 22 and 24 may be modified by a second modification on the second strand. Nucleotides are numbered for the sake of the nucleic acid of the present invention from 5' to 3' on the first strand and 3' and 5' on the second strand

The nucleotides numbered 2, 4, 6, 8, 10, 12, 14, 16, 18, 20, 22 and 24 may be modified by a modification on the first strand. The nucleotides numbered 1, 3, 5, 7, 9, 11, 13, 15, 17, 19, 21, 23 may be modified by a second modification on the first strand. The nucleotides numbered 1, 3, 5, 7, 9, 11, 13, 15, 17, 19, 21, 23 may be modified by a modification on the second strand. The nucleotides numbered 2, 4, 6, 8, 10, 12, 14, 16, 18, 20, 22 and 24 may be modified by a second modification on the second strand.

Clearly, if the first and/or the second strand are shorter than 25 nucleotides in length, such as 19 nucleotides in length, there are no nucleotides numbered 20, 21, 22, 23, 24 and 25 to be modified. The skilled person understands the description above to apply to shorter strands, accordingly.

One or more modified nucleotides on the first strand may be paired with modified nucleotides on the second strand having a common modification. One or more modified nucleotides on the first strand may be paired with modified nucleotides on the second strand having a different modification. One or more modified nucleotides on the first strand may be paired with unmodified nucleotides on the second strand. One or more modified nucleotides on the second strand may be paired with unmodified nucleotides on the first strand. In other words, the alternating nucleotides can be aligned on the two strands such as, for example, all the modifications in the alternating regions of the second strand are paired with identical modifications in the first strand or alternatively the modifications can be offset by one nucleotide with the common modifications in the alternating regions of one strand pairing with dissimilar modifications (i.e. a second or further modification) in the other strand. Another option is to have dissimilar modifications in each of the strands.

The modifications on the first strand may be shifted by one nucleotide relative to the modified nucleotides on the second strand, such that common modified nucleotides are not paired with each other.

The modification and/or modifications may each and individually be selected from the group consisting of 3'-terminal deoxy-thymine, 2'-O-methyl, a 2'-deoxy-modification, a 2'-amino-modification, a 2'-alkyl-modification, a morpholino modification, a phosphoramidate modification, 5'-phosphorothioate group modification, a 5' phosphate or 5' phosphate mimic modification and a cholesteryl derivative or a dodecanoic acid bisdecylamide group modification and/or the modified nucleotide may be any one of a locked nucleotide, an abasic nucleotide or a non-natural base comprising nucleotide. At least one modification may be 2'-O-methyl and/or at least one modification may be 2'-F. Further modifications as described herein may be present on the first and/or second strand.

Throughout the description of the invention, "same or common modification" means the same modification to any nucleotide, be that A, G, C or U modified with a group such as such as a methyl group or a fluoro group. Is it not taken to mean the same addition on the same nucleotide. For example, 2'F-dU, 2'F-dA, 2'F-dC, 2'F-dG are all considered to be the same or common modification, as are 2'-OMe-rU, 2'-OMe-rA; 2'-OMe-rC; 2'-OMe-rG. A2'F modification is a different modification to a 2'OMe modification.

Some representative modified nucleic acid sequences of the present invention are shown in the examples. These examples are meant to be representative and not limiting.

Preferably, the nucleic acid may comprise a modification and the second or further modification which are each and individually selected from the group comprising 2'-O-methyl modification and 2'-F modification. The nucleic acid may comprise a modification that is 2'-O-methyl (2'OMe) that may be a first modification, and a second modification that is 2'-F. The nucleic acid of the invention may also include a phosphorothioate modification and/or a deoxy modification which may be present in or between the terminal 1, 2 or 3 nucleotides of each or any end of each or both strands.

The nucleic acid of the invention may be conjugated to a ligand, to form a conjugate. Means for efficient delivery of oligonucleotides, in particular double stranded nucleic acids of the invention, to cells *in vivo* is important and requires specific targeting and substantial protection from the extracellular environment, particularly serum proteins. One method of achieving specific targeting is to conjugate a targeting moiety or ligand to the nucleic acid. The targeting moiety helps in targeting the nucleic acid to the required target site and there is a need to conjugate appropriate targeting moieties for the desired receptor sites for the conjugated molecules to be taken up by the cells such as by endocytosis.

For example, the Asialoglycoprotein receptor (ASGP-R) is a high capacity receptor, which is highly abundant on hepatocytes. One of the first disclosures of triantennary cluster glycosides was in US patent number US 5,885,968. Conjugates having three GalNAc ligands and comprising phosphate groups are known and are described in Dubber et al. (2003). The ASGP-R shows a 50-fold higher affinity for N-Acetyl-D-Galactosylamine (GalNAc) than D-Gal.

Hepatocytes expressing the lectin (asialoglycoprotein receptor; ASGPR), which recognizes specifically terminal β-galactosyl subunits of glycosylated proteins or other oligosaccharides (P. H. Weigel et. al., 2002,) can be used for targeting a drug to the liver by covalent coupling of galactose or galactoseamine to the drug substance (S.Ishibashi, et. al. 1994). Furthermore the binding affinity can be significantly increased by the multi-valency effect, which is achieved by the repetition of the targeting unit (E. A. L. Biessen et. al., 1995).

The ASGPR is a mediator for an active endosomal transport of terminal β-galactosyl containing glycoproteins, thus ASGPR is highly suitable for targeted delivery of drug candidates like nucleic acid, which have to be delivered into a cell (Akinc et al.).

The ligand may be attached to the nucleic acid of the invention via a linker, which may be a bivalent or trivalent or tetramer branched linker.

The saccharide, which can also be referred to as the ligand, may be selected to have an affinity for at least one type of receptor on a target cell. In particular, the receptor is on the surface of a mammalian liver cell, for example, the hepatic asialoglycoprotein receptor (ASGP-R).

The saccharide may be selected from N-acetyl galactoseamine, mannose, galactose, glucose, glucosamone and fucose. The saccharide may be N-acetyl galactoseamine (GalNAc).

"GalNAc" refers to 2-(Acetylamino)-2-deoxy-D- galactopyranose, commonly referred to in the literature as N-acetyl galactosamine. Reference to "GalNAc" or "N-acetyl galactoseamine" includes both the β- form: 2-(Acetylamino)-2-deoxy-β -D-galactopyranose and the α-form: 2-(Acetylamino)-2-deoxy-α-D- galactopyranose. Both the β-form: 2-(Acetylarnino)-2-deoxy-β-D-galactopyranose and α-form: 2-(Acetylamino)-2-deoxy-α-D-galactopyranose may be used interchangeably. Preferably, the compounds of the invention comprise the β-form, 2-(Acetylarnino)-2-deoxy-β-D-galactopyranose.

The ligand may comprise GalNAc.

The ligand may comprise a compound of formula I:

[S-X¹-P-X²]₃-A-X³-Z (I)

wherein:
S represents a saccharide;
X¹ represents C₃-C₆ alkylene or (-CH₂-CH₂-O)ₘ(-CH₂)₂- wherein m is 1, 2, or 3;
P is a modified phosphate;
X² is alkylene or an alkylene ether of the formula (-CH₂)ₙ-O-CH₂- where n = 1- 6;
A is a branching unit;
X³ represents a bridging unit;
Z is the nucleic acid molecule;
and where the linkage between X³ and Z is a phosphate or thiophosphate.

In formula I, branching unit "A" branches into three in order to accommodate the three saccharide ligands. The branching unit is covalently attached to the tethered ligands and the nucleic acid. The branching unit may comprise a branched aliphatic group comprising groups selected from alkyl, amide, disulphide, polyethylene glycol, ether, thioether and hydroxyamino groups. The branching unit may comprise groups selected from alkyl and ether groups.

The branching unit A may have a structure selected from: 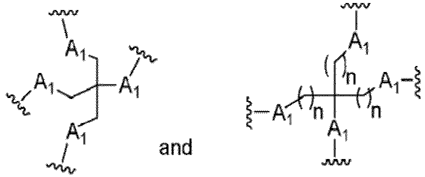 wherein each A₁ independently represents O, S, C=O or NH; and each n independently represents an integer from 1 to 20.

The branching unit may have a structure selected from: 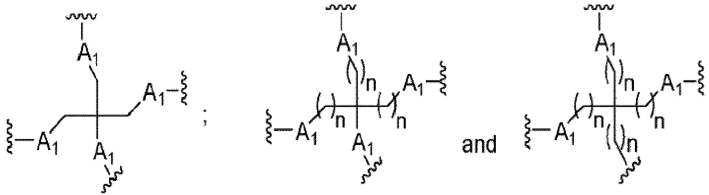 wherein each A₁ independently represents O, S, C=O or NH; and each n independently represents an integer from 1 to 20.

The branching unit may have a structure selected from: 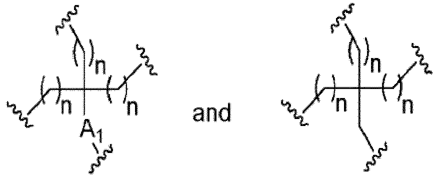 wherein A₁ is O, S, C=O or NH; and
each n independently represents an integer from 1 to 20.

The branching unit may have the structure:

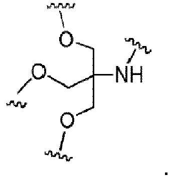

The branching unit may have the structure:

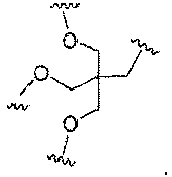

The branching unit may have the structure:

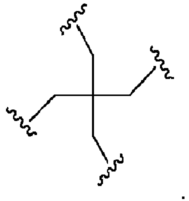

Optionally, the branching unit consists of only a carbon atom.

The "X³" portion of the compounds of formula I is a bridging unit. X³ may also be referred to as the conjugate linker. The bridging unit is linear and is covalently bound to the branching unit and the nucleic acid.

X³ may be selected from -C₁-C₂₀ alkylene-, -C₂-C₂₀ alkenylene-, an alkylene ether of formula -(C₁-C₂₀ alkylene)-O-(C₁-C₂₀ alkylene)-, -C(O)-C₁-C₂₀ alkylene-, -C₀-C₄ alkylene(Cy)C₀-C₄ alkylene- wherein Cy represents a substituted or unsubstituted 5 or 6 membered cycloalkylene, arylene, heterocyclylene or heteroarylene ring, -C₁-C₄ alkylene-NHC(O)-C₁-C₄ alkylene-, -C₁-C₄ alkylene-C(O)NH-C₁-C₄ alkylene-, -C₁-C₄ alkylene-SC(O)-C₁-C₄ alkylene-, -C₁-C₄ alkylene-C(O)S-C₁-C₄ alkylene-, -C₁-C₄ alkylene-OC(O)-C₁-C₄ alkylene-, -C₁-C₄ alkylene-C(O)O-C₁-C₄ alkylene-, and -C₁-C₆ alkylene-S-S-C₁-C₆ alkylene-.

X³ may be an alkylene ether of formula -(C₁-C₂₀ alkylene)-O-(C₁-C₂₀ alkylene)-. X³ may be an alkylene ether of formula -(C₁-C₂₀ alkylene)-O-(C₄-C₂₀ alkylene)-, wherein said (C₄-C₂₀ alkylene) is linked to Z. X³ may be selected from the group consisting of -CH₂-O-C₃H₆-, -CH₂-O-C₄H₈-, -CH₂-O-C₆H₁₂- and -CH₂-O-C₈H₁₆-, especially -CH₂-O-C₄H₈-, -CH₂-O-C₆H₁₂- and -CH₂-O-C₈H₁₆-, wherein in each case the -CH₂- group is linked to A.

The ligand may comprise a compound of formula (II):

[S-X¹-P-X²]₃-A-X³-Z (II)

wherein:
S represents a saccharide;
X¹ represents C₃-C₆ alkylene or an ethylene glycol stem (-CH₂-CH₂-O)ₘ(-CH₂)₂-wherein m is 1, 2, or 3;
P is a modified phosphate;
X² is C₁-C₈ alkylene;
A is a branching unit selected from: 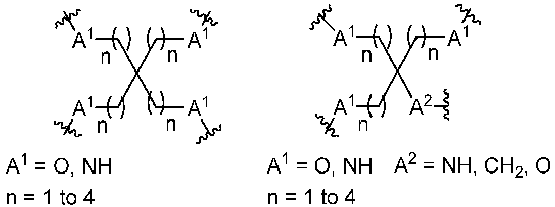
X³ is a bridging unit;
Z is the nucleic acid;
and where the linkage between X³ and Z is a phosphate or thiophosphate.

Branching unit A may have the structure:

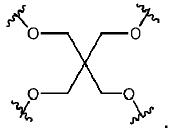

Branching unit A may have the structure:

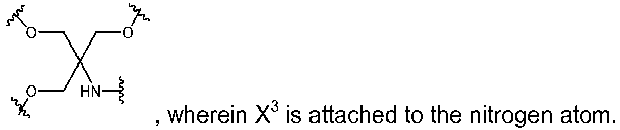

X³ may be C₁-C₂₀ alkylene. Preferably, X³ is selected from the group consisting of -C₃H₆-,-C₄H₈-, -C₆H₁₂- and -C₈H₁₆-, especially -C₄H₈-, -C₆H₁₂- and -C₈H₁₆-.

The ligand may comprise a compound of formula (III):

[S-X¹-P-X²]₃-A-X³-Z (III)

wherein:
S represents a saccharide;
X¹ represents C₃-C₆ alkylene or an ethylene glycol stem (-CH₂-CH₂-O)ₘ(-CH₂)₂-wherein m is 1, 2, or 3;
P is a modified phosphate;
X² is an alkylene ether of formula -C₃H₆-O-CH₂-;
A is a branching unit;
X³ is an alkylene ether of formula selected from the group consisting of -CH₂-O-CH₂-, -CH₂-O-C₂H₄-, -CH₂-O-C₃H₆-, -CH₂-O-C₄H₈-, -CH₂-O-C₅H-₁₀-, -CH₂-O-C₆H₁₂-, -CH₂-O-C₇H₁₄-, and -CH₂-O-C₈H₁₆-, wherein in each case the -CH₂- group is linked to A,
Z is the nucleic acid;
and wherein the linkage between X³ and Z is a phosphate or thiophosphate

The branching unit may comprise carbon. Preferably, the carbon unit is carbon.

X³ may be selected from the group consisting of -CH₂-O-C₄H₈-, -CH₂-O-C₅H₁₀-, -CH₂-O-C₆H₁₂-, -CH₂-O-C₇H₁₄-, and -CH₂-O-C₈H₁₆-. Preferably, X³ is selected from the group consisting of -CH₂-O-C₄H₈-, -CH₂-O-C₆H₁₂- and -CH₂-O-C₈H₁₆.

For any of the above aspects, P represents a modified phosphate group. P can be represented by: 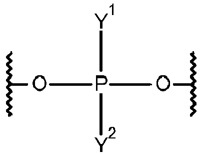 wherein Y¹ and Y² each independently represent =O, =S, -O⁻, -OH, -SH, -BH₃, -OCH₂CO₂, -OCH₂CO₂R^{x}, -OCH₂C(S)OR^{x}, and -OR^{x}, wherein R^{x} represents C₁-C₆ alkyl and wherein 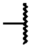 indicates attachment to the remainder of the compound.

For example, Y¹ may represent -OH and Y² may represent =O or =S; or
Y¹ may represent -O⁻ and Y² may represent =O or =S;
Y¹ may represent =O and Y² may represent -CH₃, -SH, -OR^{x}, or -BH₃
Y¹ may represent =S and Y² may represent -CH₃, OR^{x} or-SH.

It will be understood by the skilled person that in certain instances there will be delocalisation between Y¹ and Y².

Preferably, the modified phosphate group is a thiophosphate group. Thiophosphate groups include bithiophosphate (i.e. where Y¹ represents =S and Y² represents -S⁻) and monothiophosphate (i.e. where Y¹ represents -O⁻ and Y² represents =S, or where Y¹ represents =O and Y² represents -S⁻). Preferably, P is a monothiophosphate. The inventors have found that conjugates having thiophosphate groups in replacement of phosphate groups have improved potency and duration of action *in vivo.*
P may also be an ethylphosphate (i.e. where Y¹ represents =O and Y² represents OCH₂CH₃).

The saccharide, which can also be referred to as the ligand, may be selected to have an affinity for at least one type of receptor on a target cell. In particular, the receptor is on the surface of a mammalian liver cell, for example, the hepatic asialoglycoprotein receptor (ASGP-R).

For any of the above aspects, the saccharide may be selected from N-acetyl with one or more of galactosamine, mannose, galactose, glucose, glucosamine and fructose. Preferably, the saccharide is two molecules of N-acetyl galactosamine (GalNAc). The compounds of the invention may have 3 ligands which are each preferably N-acetyl galactosamine.

"GalNAc" refers to 2-(Acetylamino)-2-deoxy-D- galactopyranose, commonly referred to in the literature as N-acetyl galactosamine. Reference to "GalNAc" or "N-acetyl galactosamine" includes both the β- form: 2-(Acetylamino)-2-deoxy-β -D-galactopyranose and the α-form: 2-(Acetylamino)-2-deoxy-α-D- galactopyranose. In certain embodiments, both the β-form: 2-(Acetylarnino)-2-deoxy-β-D-galactopyranose and α-form: 2-(Acetylamino)-2-deoxy-a-D-galactopyranose may be used interchangeably. Preferably, the compounds of the invention comprise the β-form, 2-(Acetylarnino)-2-deoxy-β-D-galactopyranose. 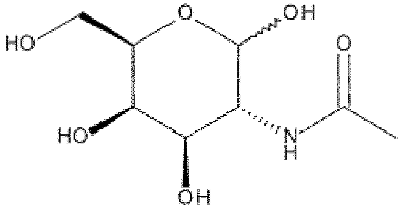 2-(Acetylamino)-2-deoxy-D-galactopyranose 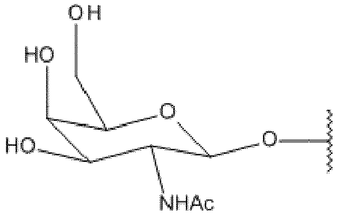 2-(Acetylamino)-2-deoxy-β-D-galactopyranose 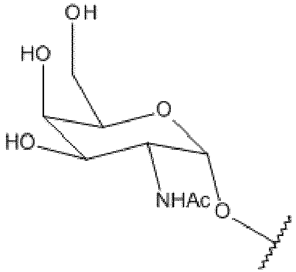 2-(Acetylamino)-2-deoxy-α-D-galactopyranose

The "X¹-P-X²" portion of the compounds of the present invention may also be referred to as the tether or linker. The linker comprises a linear group and is covalently attached to the saccharide ligand and the branching unit.

For any of the above compounds of formula (III), X¹ may be an ethylene glycol stem (-CH₂-CH₂-O)ₘ(-CH₂)₂- wherein m is 1, 2, or 3. X¹ may be (-CH₂-CH₂-O)(-CH₂)₂-. X¹ may be (-CH₂-CH₂-O)₂(-CH₂)₂-. X¹ may be (-CH₂-CH₂-O)₃(-CH₂)₂-. Preferably, X¹ is (-CH₂-CH₂-O)₂(-CH₂)₂-. Alternatively, X¹ represents C₃-C₆ alkylene. X¹ may be propylene. X¹ may be butylene. X¹ may be pentylene. X¹ may be hexylene. Preferably the alkyl is a linear alkylene. In particular, X¹ may be butylene.

For compounds of formula (III), represents an alkylene ether of formula -C₃H₆-O-CH₂-i.e. C₃ alkoxy methylene, or -CH₂CH₂CH₂OCH₂-.

The ligand may comprise or be a conjugate compound having the structure: 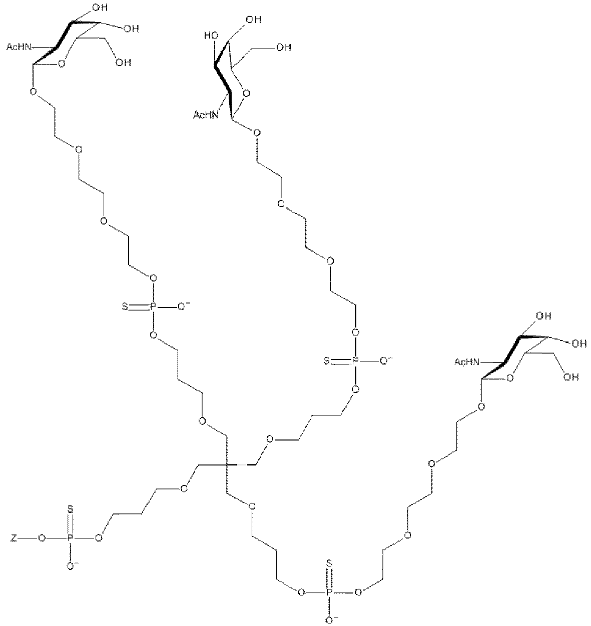 wherein Z is the nucleic acid.

The ligand may comprise or be a conjugate compound having the structure: 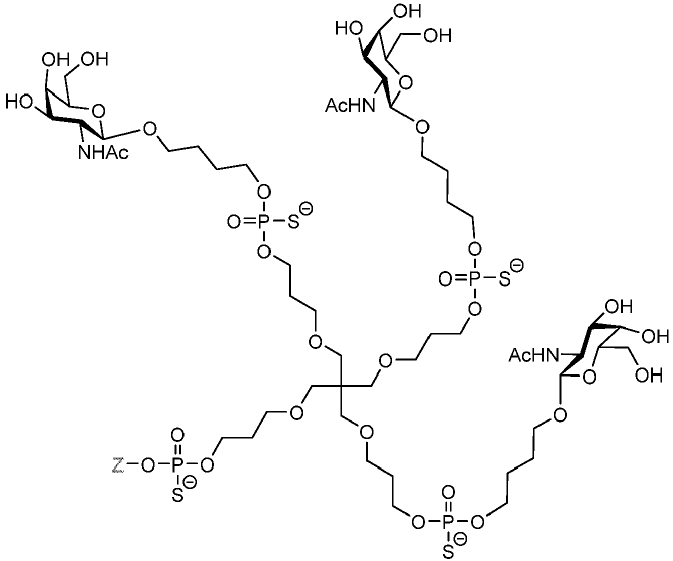 wherein Z is the nucleic acid.

The ligand may comprise or be a conjugate compound having the structure: 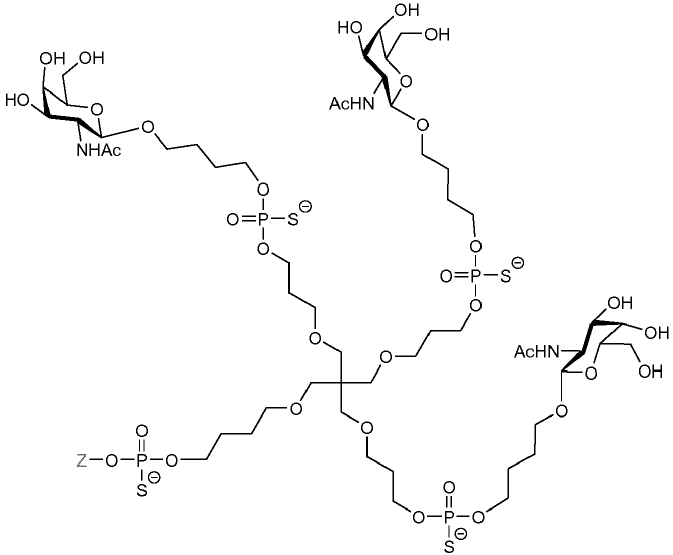 wherein Z is the nucleic acid.

The ligand may comprise or be a conjugate compound having the structure: 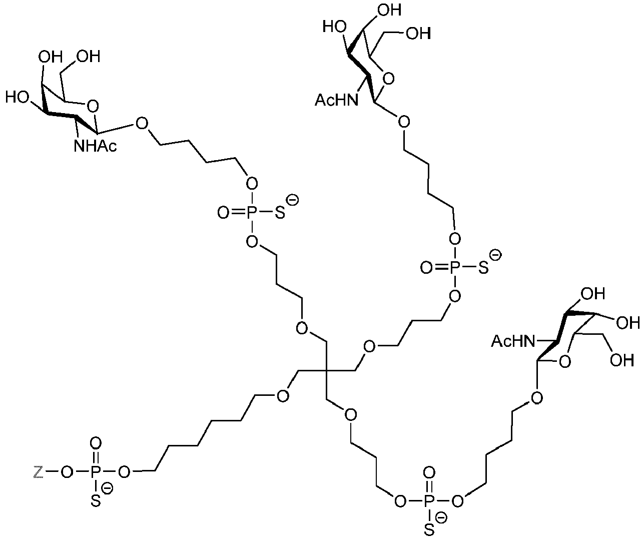 wherein Z is the nucleic acid.

The ligand may comprise or be a conjugate compound having the structure: 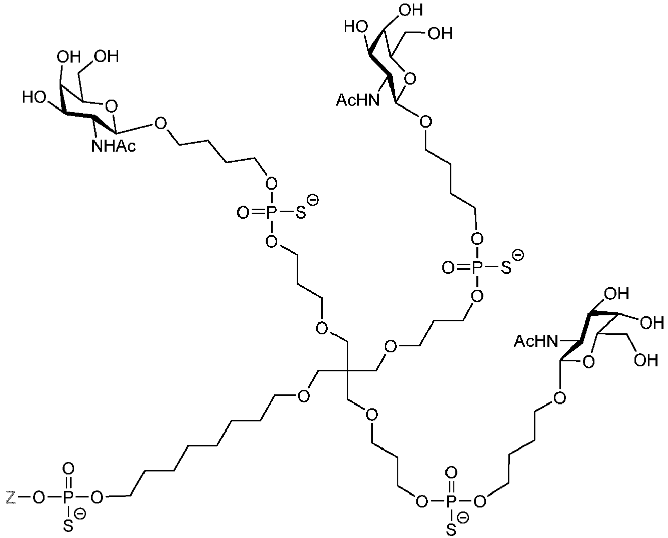 wherein Z is the nucleic acid.

The ligand may comprise or be a conjugate compound having the structure: 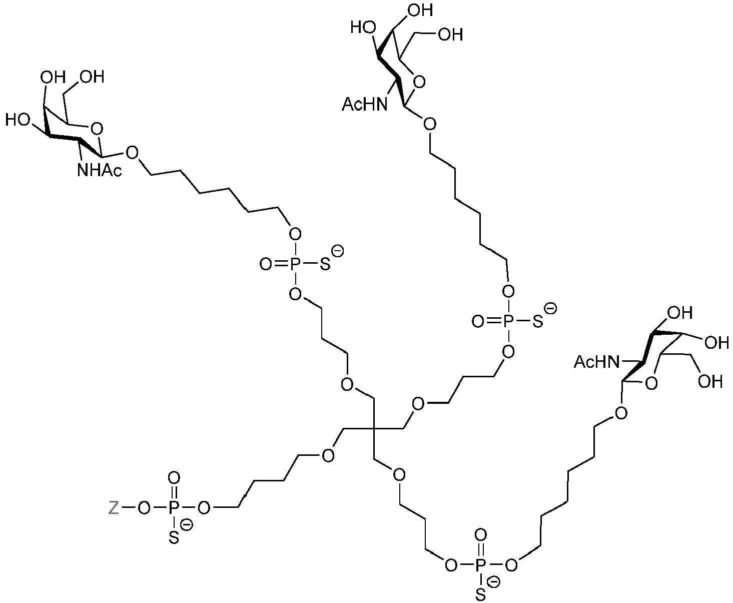 wherein Z is the nucleic acid.

The ligand may comprise or be a conjugate compound having the structure: 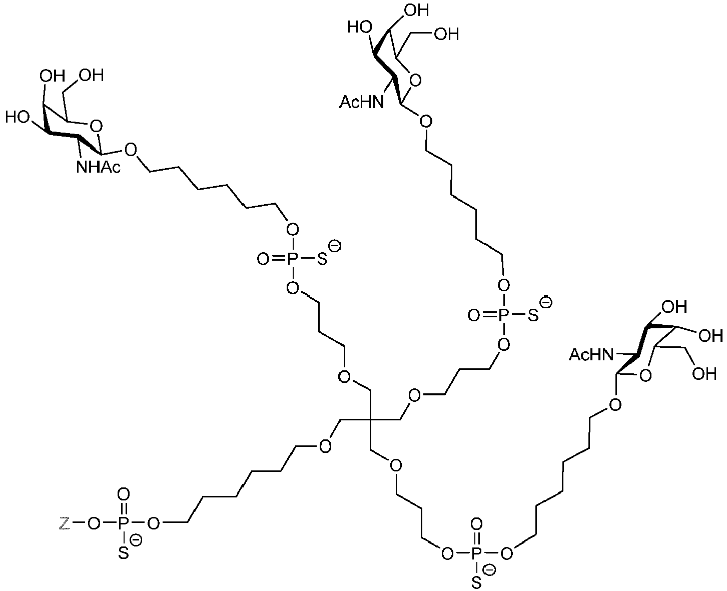 wherein Z is the nucleic acid.

The ligand may comprise or be a conjugate compound having the structure: 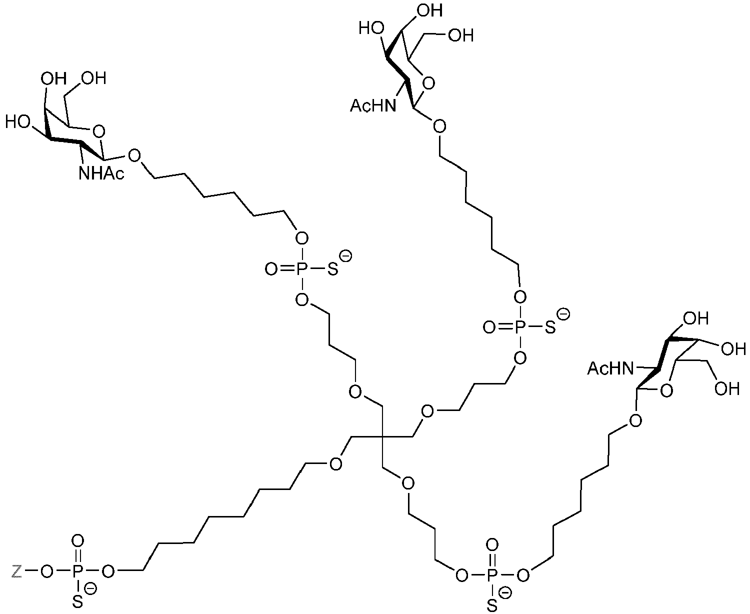 wherein Z is a nucleic acid.

The invention provides as a further aspect, a nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of a RNA transcribed from said target gene to be inhibited and wherein the terminal nucleotide at the 3' end of at least one of the first strand and the second strand is attached to the adjacent nucleotide via the 3' carbon of the terminal nucleotide and the 3' carbon of the adjacent nucleotide and/ or the terminal nucleotide at the 5' end of at least one of the first strand and the second strand is attached to the adjacent nucleotide via the 5' carbon of the terminal nucleotide and the 5' carbon of the adjacent nucleotide, and wherein the nucleic acid molecule is directly or indirectly conjugated to a ligand via a linker.

The nucleic acid may be conjugated to a ligand as herein described via a linker, as herein described. The nucleotides of the first and/or second strand may be modified, as herein described.

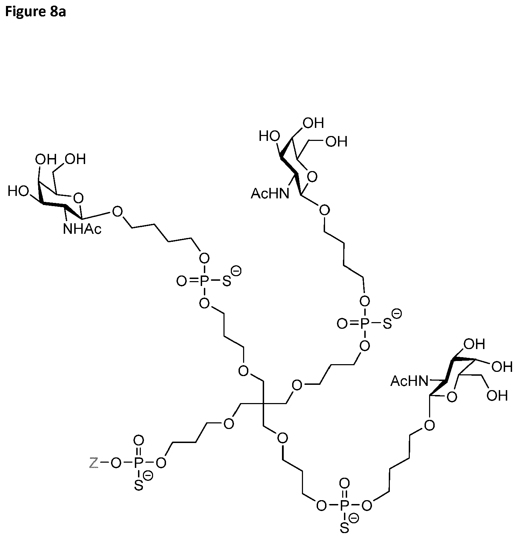

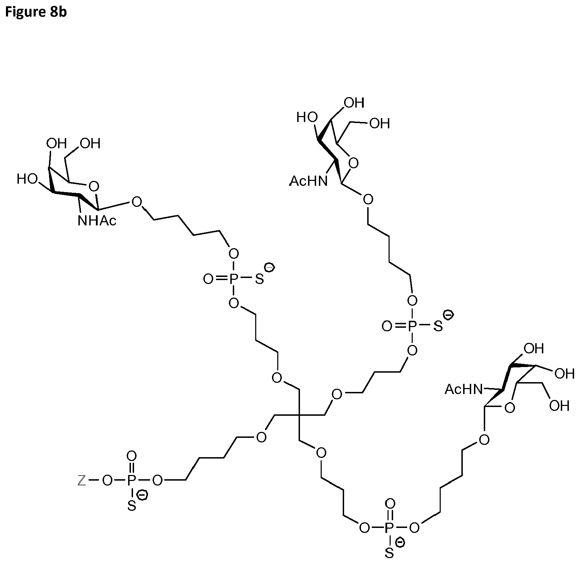

The ligand may be GalNac and be attached via a linker, which together may be of the structure set out in Figure 8a or Figure 8b.

The nucleic acid as described herein may be formulated with a lipid in the form of a liposome. Such a formulation may be described in the art as a lipoplex. The formulation with a lipid/liposome may be used to assist with delivery of the nucleic acid of the invention to the target cells. The lipid delivery system herein described may be used as an alternative to a conjugated ligand. The modifications herein described may be present when using the nucleic acid of the invention with a lipid delivery system or with a ligand conjugate delivery system.

Such a lipoplex may comprise a lipid formulation comprising:
i) a cationic lipid, or a pharmaceutically acceptable salt thereof;
ii) a steroid;
iii) a phosphatidylethanolamine phospholipid;
iv) a PEGylated lipid.

The cationic lipid may be an amino cationic lipid.

The cationic lipid may have the formula (I): 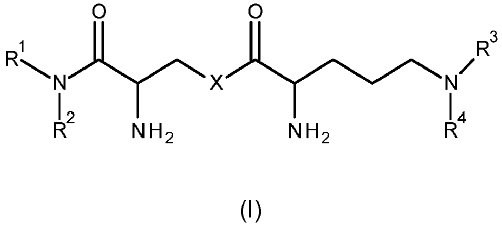 or a pharmaceutically acceptable salt thereof, wherein:
X represents O, S or NH;
R¹ and R² each independently represents a C₄-C₂₂ linear or branched alkyl chain or a C₄-C₂₂ linear or branched alkenyl chain with one or more double bonds, wherein the alkyl or alkenyl chain optionally contains an intervening ester, amide or disulfide;
when X represents S or NH, R³ and R⁴ each independently represent hydrogen, methyl, ethyl, a mono- or polyamine moiety, or R³ and R⁴ together form a heterocyclyl ring; when X represents O, R³ and R⁴ each independently represent hydrogen, methyl, ethyl, a mono- or polyamine moiety, or R³ and R⁴ together form a heterocyclyl ring, or R³ represents hydrogen and R⁴ represents C(NH)(NH₂).

The cationic lipid may have the formula (IA): 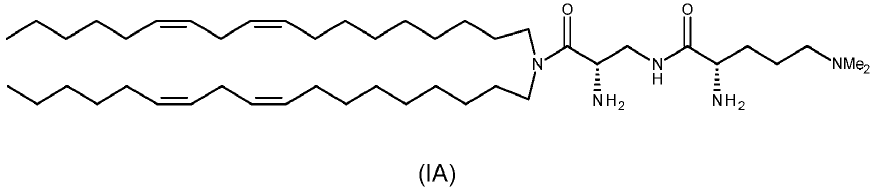 or a pharmaceutically acceptable salt thereof.

The cationic lipid may have the formula (IB): 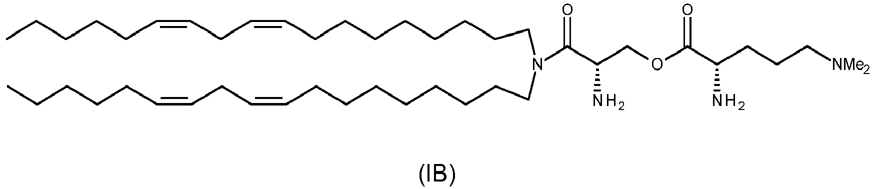 or a pharmaceutically acceptable salt thereof.

The content of the cationic lipid component may be from about 55 mol% to about 65 mol% of the overall lipid content of the formulation. In particular, the cationic lipid component is about 59 mol% of the overall lipid content of the formulation.

The formulations further comprise a steroid. the steroid may be cholesterol. The content of the steroid may be from about 26 mol% to about 35 mol% of the overall lipid content of the lipid formulation. More particularly, the content of steroid may be about 30 mol% of the overall lipid content of the lipid formulation.

The phosphatidylethanolamine phospholipid may be selected from group consisting of 1,2-diphytanoyl-sn-glycero-3-phosphoethanolamine (DPhyPE), 1,2-dioleoyl-sn-glycero-3-phosphoethanolamine (DOPE), 1,2-distearoyl-sn-glycero-3-phosphoethanolamine (DSPE), 1,2-Dilauroyl-sn-glycero-3-phosphoethanolamine (DLPE), 1,2-Dimyristoyl-sn-glycero-3-phosphoethanolamine (DMPE), 1,2-Dipalmitoyl-sn-glycero-3-phosphoethanolamine (DPPE), 1,2-Dilinoleoyl-sn-glycero-3-phosphoethanolamine (DLoPE), 1-Palmitoyl-2-oleoyl-sn-glycero-3-phosphoethanolamine (POPE), 1,2-Dierucoyl-sn-glycero-3-phosphoethanolamine (DEPE), 1,2-Disqualeoyl-sn-glycero-3-phosphoethanolamine (DSQPE) and 1-Stearoyl-2-linoleoyl-sn-glycero-3-phosphoethanolamine (SLPE). The content of the phospholipid may be about 10 mol% of the overall lipid content of the formulation.

The PEGylated lipid may be selected from the group consisting of 1,2-dimyristoyl-sn-glycerol, methoxypolyethylene glycol (DMG-PEG) and C16-Ceramide-PEG. The content of the PEGylated lipid may be about 1 to 5 mol% of the overall lipid content of the formulation.

The content of the cationic lipid component in the formulation may be from about 55 mol% to about 65 mol% of the overall lipid content of the lipid formulation, preferably about 59 mol% of the overall lipid content of the lipid formulation.

The formulation may have a molar ratio of the components of i):ii): iii): iv) selected from 55:34:10:1; 56:33:10:1; 57:32:10:1; 58:31:10:1; 59:30:10:1; 60:29:10:1; 61:28:10:1; 62:27:10:1; 63:26:10:1; 64:25:10:1; and 65:24:10:1.

The formulation may comprise a cationic lipid having the structure 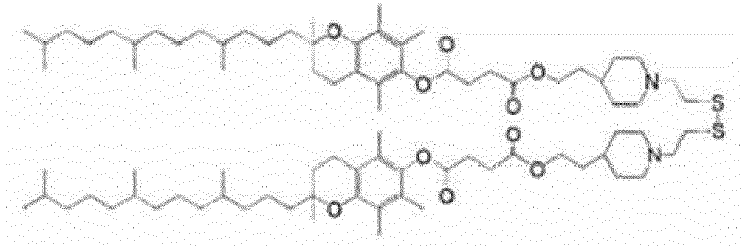 a steroid having the structure 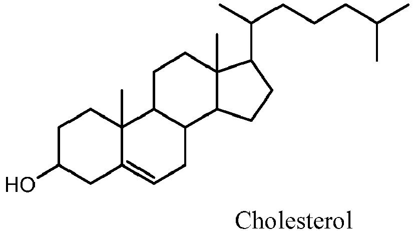 a phosphatidylethanolamine phospholipid having the structure 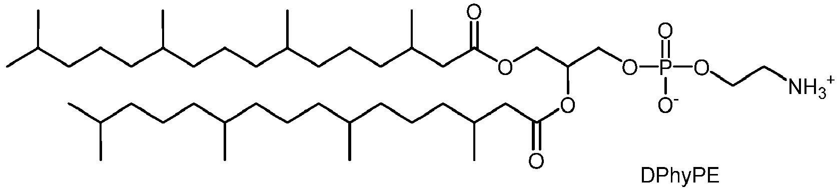 and a PEGylated lipid having the structure

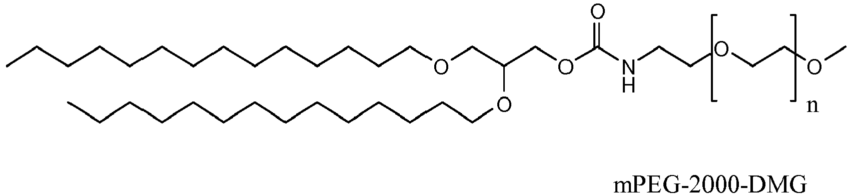

The present invention also provides pharmaceutical compositions comprising the nucleic acid of the invention. The pharmaceutical compositions may be used as medicaments or as diagnostic agents, alone or in combination with other agents. For example, one or more nucleic acid conjugates of the invention can be combined with a delivery vehicle (e.g., liposomes) and excipients, such as carriers, diluents. Other agents such as preservatives and stabilizers can also be added. Methods for the delivery of nucleic acids are known in the art and within the knowledge of the person skilled in the art.

The nucleic acid conjugates of the present invention can also be administered in combination with other therapeutic compounds, either administrated separately or simultaneously, e.g., as a combined unit dose. The invention also includes a pharmaceutical composition comprising one or more nucleic acid conjugates according to the present invention in a physiologically/pharmaceutically acceptable excipient, such as a stabilizer, preservative, diluent, buffer, and the like.

Dosage levels for the medicament and pharmaceutical compositions of the invention can be determined by those skilled in the art by routine experimentation. In one embodiment, a unit dose may contain between about 0.01 mg/kg and about 100 mg/kg body weight of nucleic acid. Alternatively, the dose can be from 10 mg/kg to 25 mg/kg body weight, or 1 mg/kg to 10 mg/kg body weight, or 0.05 mg/kg to 5 mg/kg body weight, or 0.1 mg/kg to 5 mg/kg body weight, or 0.1 mg/kg to1 mg/kg body weight, or 0.1 mg/kg to 0.5 mg/kg body weight, or 0.5 mg/kg to 1 mg/kg body weight.

The pharmaceutical composition may be a sterile injectable aqueous suspension or solution, or in a lyophilized form. In one embodiment, the pharmaceutical composition may comprise lyophilized lipoplexes or an aqueous suspension of lipoplexes. The lipoplexes preferably comprises a nucleic acid of the present invention. Such lipoplexes may be used to deliver the nucleic acid of the invention to a target cell either in vitro or in vivo.

The pharmaceutical compositions and medicaments of the present invention may be administered to a mammalian subject in a pharmaceutically effective dose. The mammal may be selected from humans, dogs, cats, horses, cattle, pig, goat, sheep, mouse, rat, hamster and guinea pig.

A further aspect of the invention relates to a nucleic acid of the invention or the pharmaceutical composition comprising the nucleic acid of the invention for use in the treatment of a disease or disorder. The invention includes a pharmaceutical composition comprising one or more RNAi molecules according to the present invention in a physiologically/ pharmaceutically acceptable excipient, such as a stabiliser, preservative, diluent, buffer and the like.

The pharmaceutical composition may be a sterile injectable aqueous suspension or solution, or in a lyophilised form.

The nucleic acid described herein may be capable of inhibiting the expression of a target gene in a cell. The nucleic acid described herein may be capable of partially inhibiting the expression of a target gene in a cell. Inhibition may be complete, i.e. 0% of the expression level of target gene expression in the absence of the nucleic acid of the invention. Inhibition of target gene expression may be partial, i.e. it may be 10%, 20%, 30%, 40%, 50%, 60%, 70%, 75%, 80%, 85%, 90%, 95% of target gene expression in the absence of a nucleic acid of the invention. Inhibition may last 4 weeks, 5 weeks, 6 weeks, 7 weeks, 8 weeks, 10 weeks, 11 weeks, 12 weeks, 13 weeks, 14 weeks or up to 3 months, when used in a subject, such as a human subject. The nucleic acid or composition comprising the nucleic acid composition may be for use once, every week, every two weeks, every three weeks, every four weeks, every five weeks, every six weeks, every seven weeks, or every eight weeks. The nucleic acid may be for use subcutaneously or intravenously.

In cells and/or subjects treated with or receiving the nucleic acid of the present invention, the target gene expression may be inhibited compared to untreated cells and/or subjects by at least about 30%, 35%, 40%, 45%, 50%, 55%, 60%, 65%, 70%, 75%, 80%, 85%, 90%, 95%, 98%, or 100%. The level of inhibition may allow treatment of a disease associated with target gene expression or overexpression, or may allow further investigation into the functions of the target gene product.

A further aspect of the invention relates to nucleic acid of the invention in the manufacture of a medicament for treating a disease or disorder.

Also included in the invention is a method of treating a disease or disorder comprising administration of a pharmaceutical composition comprising an nucleic acid as described herein, to an individual in need of treatment. The nucleic acid composition may be administered twice every week, once every week, every two weeks, every three weeks, every four weeks, every five weeks, every six weeks, every seven weeks, or every eight weeks. The nucleic acid may be administered to the subject subcutaneously or intravenously.

The nucleic acid of the present invention can also be administered or for use in combination with other therapeutic compounds, either administered separately or simultaneously, e.g. as a combined unit dose.

The nucleic acid of the present invention can be produced using routine methods in the art including chemically synthesis or expressing the nucleic acid either in vitro (e.g., run off transcription) or in vivo. For example, using solid phase chemical synthesis or using an expression vector. In one embodiment, the expression vector can produce the nucleic acid of the invention in a target cell. Methods for the synthesis of the nucleic acid described herein are known to persons skilled in the art.

The invention will now be described with reference to the following non-limiting figures and examples in which:
Figure 1 shows the 3'-3' linkage used to form an inverted nucleotide;
Figure 2 shows schematic representations of TMP70, TMP71-74 and TMP75-78;
Figures 3 and 4 show the serum stability of ivR-modified siRNAs;
Figure 5 shows the knockdown activity of ivR-modified siRNAs in vitro at a concentration of 0.1 and 1 nM siRNA;
Figure 6 shows the knockdown activity of ivR-modified siRNAs in vitro at a concentration of 5 nM to 0.00016 nM siRNA; and
Figure 7 shows the modification information of TMPRSS6 hcm-9.
Figure 8a and 8b show the structure of the GalNac, gen3 and gen4, respectively, to which the nucleotides were conjugated.

### Examples

### Example 1

siRNA modification: using inverted nucleotides.

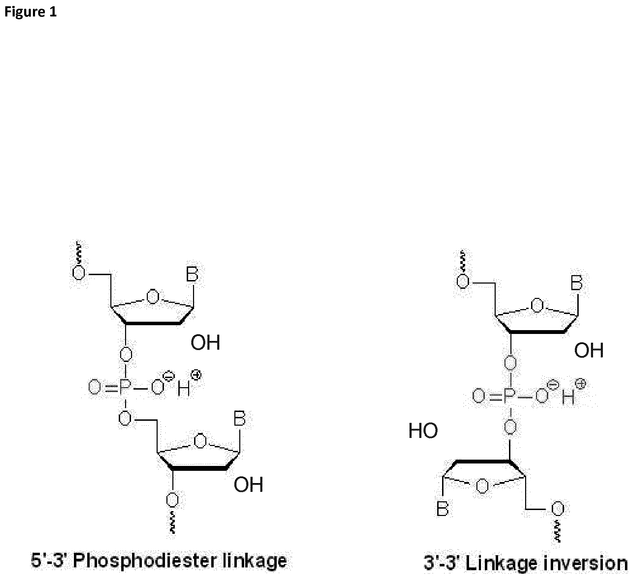

The terminal nucleotide at the 3' end of an oligonucleotide strand can be attached to the adjacent nucleotide via the 3' carbon of the terminal nucleotide and the 3' carbon of the adjacent nucleotide to form an inverted nucleotide. Likewise, the terminal nucleotide at the 5' end of a oligonucleotide strand can be attached to the adjacent nucleotide via the 5' carbon of the terminal nucleotide and the 5' carbon of the adjacent nucleotide to form an inverted nucleotide. The 3'-3' linkage is set out in Figure 1.

### Example 2

siRNA modification: synthesis of siRNA with inverted nucleotides.

All Oligonucleotides were either obtained from a commercial oligonucleotide manufacturer (Eurogentech, Belgium) or synthesized on an AKTA oligopilot synthesizer using standard phosphoramidite chemistry. Commercially available solid support and 2'0-Methyl RNA phosphoramidtes, 2'Fluoro DNA phosphoramidites (all standard protection) and commercially available long trebler phosphoramidite (Glen research) were used. Synthesis was performed using 0.1 M solutions of the phosphoramidite in dry acetonitrile and benzylthiotetrazole (BTT) was used as activator (0.3M in acetonitrile). All other reagents were commercially available standard reagents.

Conjugation of the GalNac synthon (ST23) was achieved by coupling of the respective phosphoramidite to the 5'end of the oligochain under standard phosphoramidite coupling conditions. Phosphorothioates were introduced using standard commercially available thiolation reagents (EDITH, Link technologies).

The single strands were cleaved off the CPG by using Methylamine and the resulting crude oligonucleotide was purified by lonexchange chromatography (Resource Q, 6mL, GE Healthcare) on a AKTA Pure HPLC System using a Sodium chloride gradient. Product containing fractions were pooled, desalted on a size exclusion column (Zetadex, EMP Biotech) and lyophilised.

For Duplexation, equimolar amounts of the respective single strands were dissolved in water and heated to 80°C for 5min. After cooling the resulting Duplex was lyophilised.

Sequences are shown in Table 1 and are all TMPRSS6 hcm-9.

**Table 1**

| **Duplex ID** | **strand ID** | **SEQ ID No** | **sequence and chemistry (5'- 3')** |
|---|---|---|---|
| TMP01 | TMPJH01A | 1 | AACCAGAAGAAGCAGGUGA |
| | TMPJH01B | 2 | UCACCUGCUUCUUCUGGUU |
| TMP70 | TMPJH40A | 3 | AACCAGAAGAAGCAGGUGA |
| | TMPJH40B | 4 | UCACCUGCUUUUCUGGUU |
| TMP71 | TMPJH41A | 5 | AACCAGAAGAAGCAGGUGAA* |
| | TMPJH41B | 6 | UCACCUGCUUCUUCUGGUUA* |
| TMP72 | TMPJH42A | 7 | AACCAGAAGAAGCAGGUGAU* |
| | TMPJH42B | 8 | UCACCUGCUUCUUCUGGUUU* |
| TMP73 | TMPJH43A | 9 | AACCAGAAGAAGCAGGUGAC* |
| | TMPJH43B | 10 | UCACCUGCUUCUUCUGGUUC* |
| TMP74 | TMPJH44A | 11 | AACCAGAAGAAGCAGGUGAG* |
| | TMPJH44B | 12 | UCACCUGCUUCUUCUGGUUG* |
| TMP75 | TMPJH45A | 13 | AACCAGAAGAAGCAGGUGAA* |
| | TMPJH45B | 14 | UCACCUGCUUCUUCUGGUUA* |
| TMP76 | TMPJH46A | 15 | AACCAGAAGAAGCAGGUGAU* |
| | TMPJH46B | 16 | UCACCUGCUUCUUCUGGUUU* |
| TMP77 | TMPJH47A | 17 | AACCAGAAGAAGCAGGUGAC* |
| | TMPJH47B | 18 | UCACCUGCUUCUUCUGGUUC* |
| TMP78 | TMPJH48A | 19 | AACCAGAAGAAGCAGGUGAG* |
| | TMPJH48B | 20 | UCACCUGCUUCUUCUGGUUG* |

| | | | |
|---|---|---|---|
| * = inverted RNA (3'-3' linkage) all sequences are TMPRSS6 hcm-9 | | | |

An example relates to the TMPRSS6 gene and its product, Matriptase-2 (MT-2). MT-2 is a type II transmembrane serine protease that plays a critical role in the regulation of iron homeostasis. MT-2 is a negative regulator for gene expression of the peptide hormone hepcidin. Hepcidin is predominantly produced and secreted by the liver. Hepcidin regulates iron balance in the body by acting as a negative regulator of gastro-intestinal iron absorption and recycling of iron from hem by macrophages. Upregulation of hepcidin leads to a reduction in iron availability within the body (McDonald et al, American Journal of Physiology, 2015, vol 08 no. 7, C539-C547). Hepcidin levels are low in patients with iron overload. Therefore a possible target for reducing iron overload is to increase Hepcidin by silencing TMPRSS6 gene expression in the liver.

TMP70 has two phosphorothioate at every end (5'and 3' of both strands) and alternating 2' O-methyl and 2' fluoro modifications on both strands (such that every 2'O-Me modified nucleotide on the first strand is paired with a 2'F modified nucleotide on the second strand).

TMP71-74 each has two phosphorothioate at every end and alternating 2' O-methyl and 2' fluoro modifications and one additional inverted nucleotide (A, U, C, G) at the 3'-ends and is phosphodiester linked.

TMP75-78 has two phosphorothioate at the 5'-ends, no phosphorothioate at the 3'-ends, one additional inverted nucleotide (A, U, C, G) at 3'-ends and is phosphodiester linked.

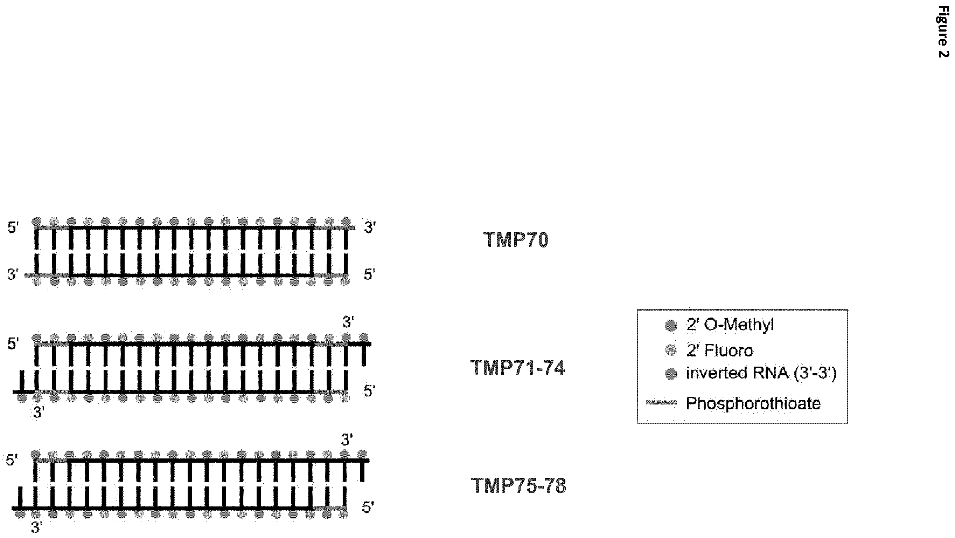

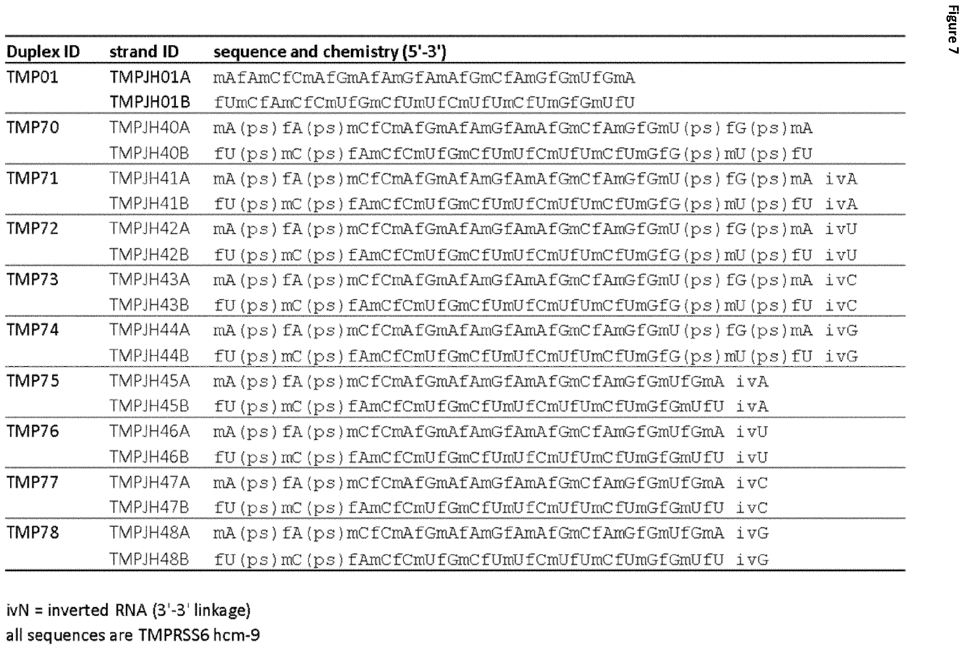

These siRNA molecules are schematically shown in Figure 2. Modification information is shown in Figure 7.

### Example 3

Serum stability of ivR-modified siRNAs was tested. TMP75 (which includes an inverted A) and TMP78 (which includes an inverted G) are more stable than TMP70.

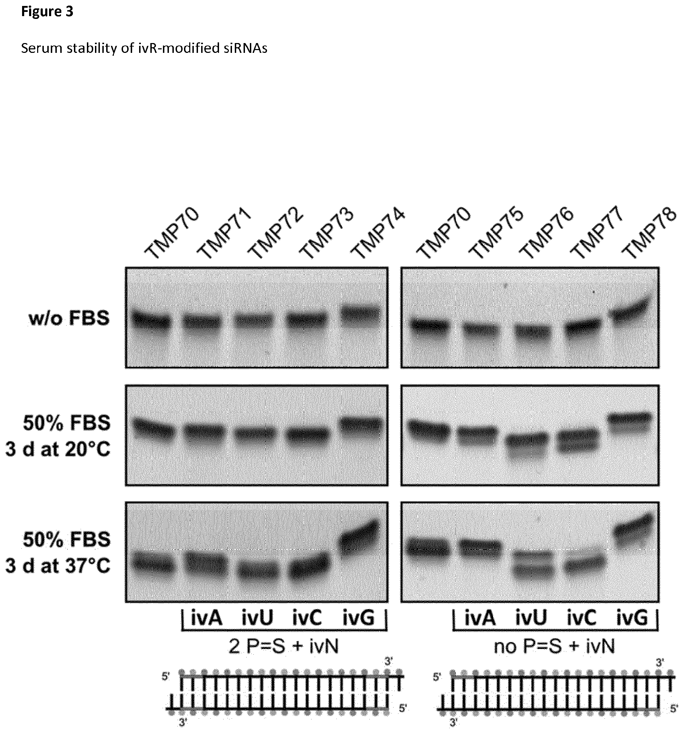

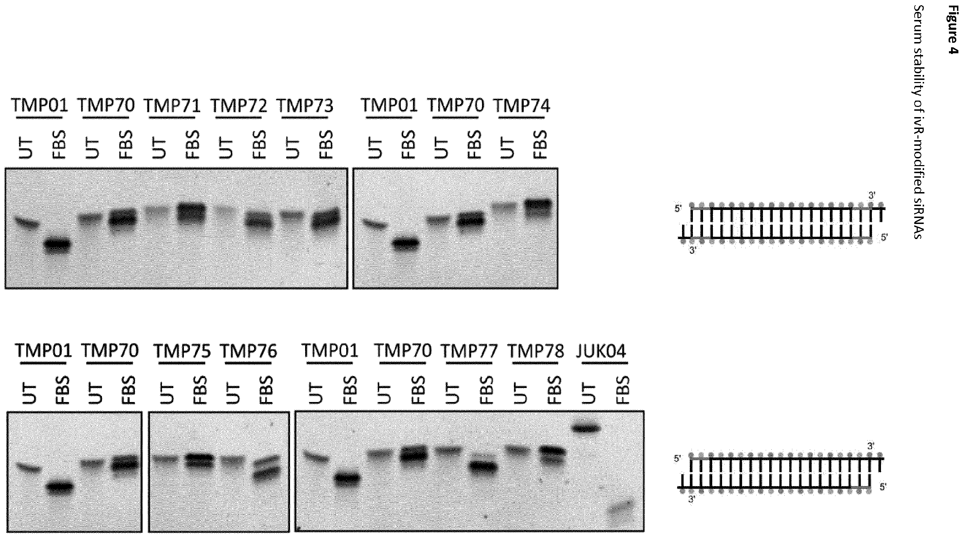

Data are shown in Figures 3 and 4.

### Example 4

Knockdown activity of ivR-modified siRNAs in vitro was tested. A Hep3B cell line was seeded at a density of 150,000 cells per 6-well. Experimental conditions: 0.1 and 1 nM siRNA, 1 µg/ml Atufect, lysis 48 hpt. All variants were found to be equally active in vitro.

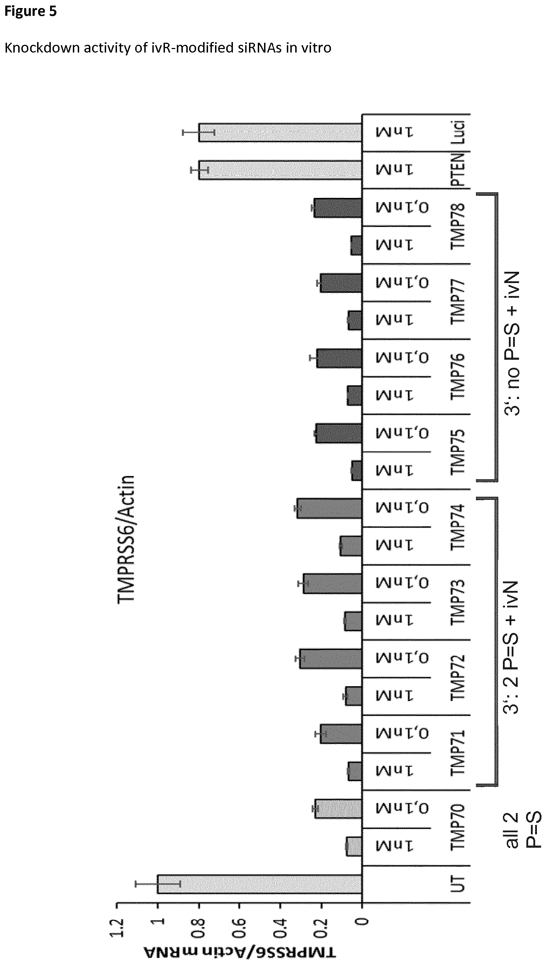

Data are shown in Figure 5.

### Example 5

Knockdown activity of ivR-modified siRNAs in vitro was tested. Hep3B cell line was seeded at a density of 150,000 cells per 6-well. Experimental condistions : 5 nM → 0.00016 nM siRNA, 1 µg/ml Atufect, lysis 48 hpt.

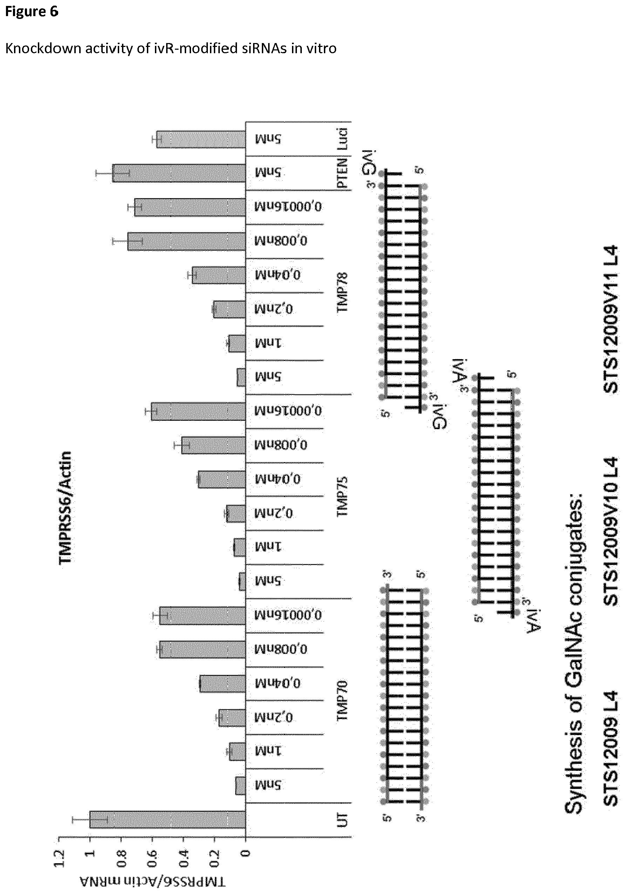

Data are shown in Figure 6.

## Claims

1. A nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of RNA transcribed from said target gene to be inhibited and wherein the terminal nucleotide at the 3' end of at least one of the first strand and the second strand is attached to the adjacent nucleotide via the 3' carbon of the terminal nucleotide and the 3' carbon of the adjacent nucleotide and/ or the terminal nucleotide at the 5' end of at least one of the first strand and the second strand is attached to the adjacent nucleotide via the 5' carbon of the terminal nucleotide and the 5' carbon of the adjacent nucleotide.

2. A nucleic acid according to claim 1, wherein 3' and/or 5' terminal nucleotides are attached to the adjacent nucleotides via a phosphate group.

3. A nucleic acid of claim 1 or claim 2, wherein the first strand and the second strand are separate strands.

4. A nucleic acid of claim 1 or claim 2, comprising a single strand that comprises the first strand and the second strand.

5. A nucleic acid according to any of claims 1 to 4, wherein said first strand and/or said second strand are each from 17-35 nucleotides in length.

6. A nucleic acid of any of claims 1 to 5, wherein the at least one duplex region consists of 19-25 nucleotide base pairs.

7. A nucleic acid of any preceding claim, which
a) is blunt ended at both ends; or
b) has an overhang at one end and a blunt end at the other; or
c) has an overhang at both ends.

8. A nucleic acid according to any preceding claim, wherein one or more nucleotides on the first and / or second strand are modified, to form modified nucleotides.

9. A nucleic acid of claim 8, wherein one or more of the odd numbered nucleotides of the first strand are modified.

10. A nucleic acid according to claim 9, wherein one or more of the even numbered nucleotides of the first strand are modified by at least a second modification, wherein the at least second modification is different from the modification of claim 9.

11. A nucleic acid of claim 10, wherein at least one of the one or more modified even numbered nucleotides is adjacent to at least one of the one or more modified odd numbered nucleotides.

12. A nucleic acid of any one of claims 9 to 11, wherein a plurality of odd numbered nucleotides are modified.

13. A nucleic acid of any one of claims 10 to 12, wherein a plurality of even numbered nucleotides are modified by a second modification.

14. A nucleic acid of any of claims 8 to 13, wherein the first strand comprises adjacent nucleotides that are modified by a common modification.

15. A nucleic acid of any of claims 9 to 14, wherein the first strand comprises adjacent nucleotides that are modified by a second modification that is different to the modification of claim 9.

16. A nucleic acid of any of claims 9 to 15, wherein one or more of the odd numbered nucleotides of the second strand are modified by a modification that is different to the modification of claim 9.

17. A nucleic acid according to any of claims 9 to 15, wherein one or more of the even numbered nucleotides of the second strand are modified by the modification of claim 9.

18. A nucleic acid of claim 16 or 17, wherein at least one of the one or more modified even numbered nucleotides of the second strand is adjacent to the one or more modified odd numbered nucleotides.

19. A nucleic acid of any of claims 16 to 18, wherein a plurality of odd numbered nucleotides of the second strand are modified by a common modification.

20. A nucleic acid of any of claims 16 to 19, wherein a plurality of even numbered nucleotides are modified by a modification according to claim 9.

21. A nucleic acid of any of claims 16 to 20, wherein a plurality of odd numbered nucleotides are modified by a second modification, wherein the second modification is different from the modification of claim 9.

22. A nucleic acid of any of claims 16 to 21, wherein the second strand comprises adjacent nucleotides that are modified by a common modification.

23. A nucleic acid of any of claims 16 to 22, wherein the second strand comprises adjacent nucleotides that are modified by a second modification that is different from the modification of claim 9.

24. A nucleic acid according to any one of claims 8 to 23, wherein each of the odd numbered nucleotides in the first strand and each of the even numbered nucleotides in the second strand are modified with a common modification.

25. A nucleic acid of any one of claims 9 to 24, wherein each of the even numbered nucleotides are modified in the first strand with a second modification and each of the odd numbered nucleotides are modified in the second strand with a second modification.

26. A nucleic acid according to any one of claims 16 to 25, wherein the modified nucleotides of the first strand are shifted by at least one nucleotide relative to the unmodified or differently modified nucleotides of the second strand.

27. A nucleic acid of any one of claims 1 to 26, wherein the first strand comprises a sequence selected form the group consisting of SEQ ID NO:s 1 to 5.

28. A nucleic acid of any one of claims 1 to 26, wherein the second strand comprises a sequence selected from the group consisting to SEQ ID NO:s 6 to 10.

29. A nucleic acid according to any one of claims 8 to 28, wherein the modification and/or modifications are each and individually selected from the group consisting of 3'-terminal deoxy-thymine, 2'-O-methyl, a 2'-deoxy-modification, a 2'-amino-modification, a 2'-alkyl-modification, a morpholino modification, a phosphoramidate modification, 5'-phosphorothioate group modification, a 5' phosphate or 5' phosphate mimic modification and a cholesteryl derivative or a dodecanoic acid bisdecylamide group modification.

30. A nucleic acid according to any one of claims 8 to 29, wherein the modification is any one of a locked nucleotide, an abasic nucleotide or a non-natural base comprising nucleotide.

31. A nucleic acid according to any one of claims 8 to 30, wherein at least one modification is 2'-O-methyl.

32. A nucleic acid according to any one of claims 8 to 31, wherein at least one modification is 2'-F.

33. A nucleic acid according to any one of claims 1 to 32, wherein the terminal nucleotide at the 3' end of at least one of the first strand and the second strand that is attached to the adjacent nucleotide via the 3' carbon of the terminal nucleotide and the 3' carbon of the adjacent nucleotide and/ or the terminal nucleotide at the 5' end of at least one of the first strand and the second strand that is attached to the adjacent nucleotide via the 5' carbon of the terminal nucleotide and the 5' carbon of the adjacent nucleotide is a purine.

34. A nucleic acid according to any one of claims 1 to 33, further comprising a ligand.

35. A nucleic acid according to any one of claims 1 to 34, comprising a phosphorothioate linkage between the terminal one, two or three 3' nucleotides and/or 5' nucleotides of the first and/or the second strand.

36. A nucleic acid according to any one of claims 1 to 35, comprising two phosphorothioate linkage between each of the three terminal 3' and between each of the three terminal 5' nucleotides on the first strand, and two phosphorothioate linkages between the three terminal nucleotides of the 3' end of the second strand.

37. A nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of RNA transcribed from said target gene to be inhibited and wherein the terminal nucleotide at the 3' end of at least one of the first strand and the second strand is attached to the adjacent nucleotide via the 3' carbon of the terminal nucleotide and the 3' carbon of the adjacent nucleotide and/ or the terminal nucleotide at the 5' end of at least one of the first strand and the second strand is attached to the adjacent nucleotide via the 5' carbon of the terminal nucleotide and the 5' carbon of the adjacent nucleotide, and wherein the nucleic acid molecule is directly or indirectly conjugated to a ligand via a linker.

38. A nucleic acid according to any of claims 34 to 37, wherein the ligand comprises one or more GalNac ligands and derivatives thereof.

39. A nucleic acid according to any of claims 34 to 38, wherein the ligand is directly or indirectly conjugated to a nucleic acid as defined in any preceding claims by a bivalent or trivalent branched linker.

40. A nucleic acid of claim 37, wherein the nucleotides are modified as defined in any preceding claims.

41. A nucleic acid of any preceding claim, wherein the ligand comprises the formula I:
[S-X¹-P-X²]₃-A-X³-Z (I)
wherein:
S represents a saccharide, wherein the saccharide is N-acetyl galactosamine;
X¹ represents C₃-C₆ alkylene or (-CH₂-CH₂-O)ₘ(-CH₂)₂- wherein m is 1, 2, or 3;
P is a modified phosphate;
X² is alkylene or an alkylene ether of the formula (-CH₂)ₙ-O-CH₂- where n = 1- 6;
A is a branching unit;
X³ represents a bridging unit;
Z is the nucleic acid;
and where the linkage between X³ and Z is a phosphate or thiophosphate.

42. A nucleic acid of any preceding claim, wherein the ligand is any one of:

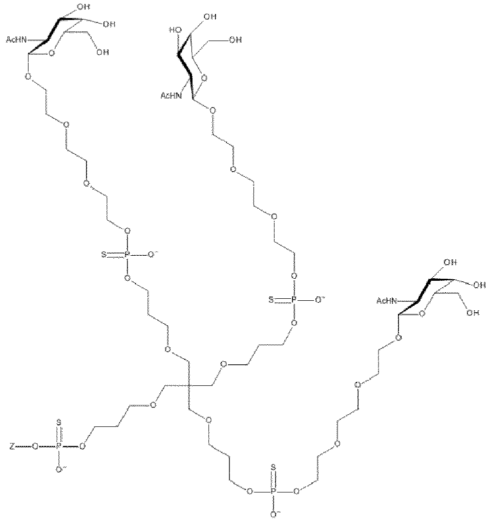

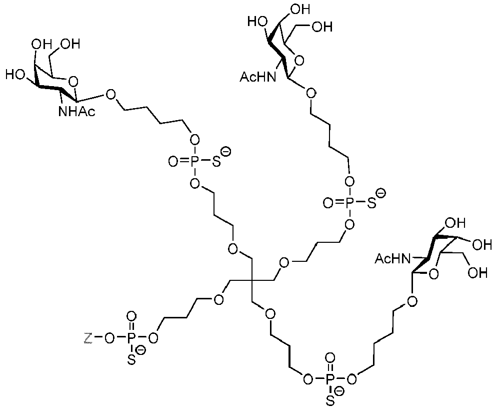

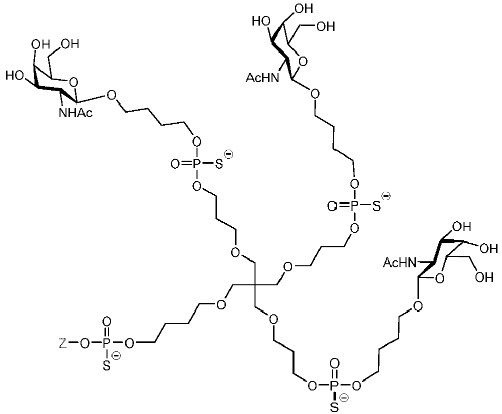

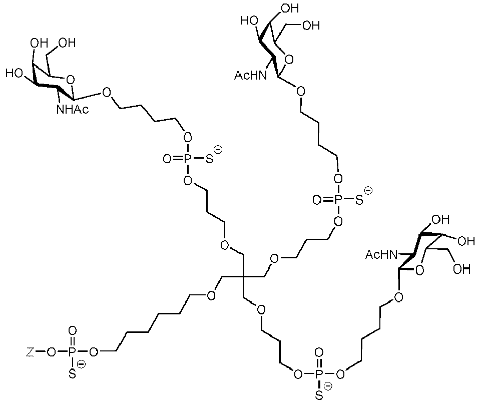

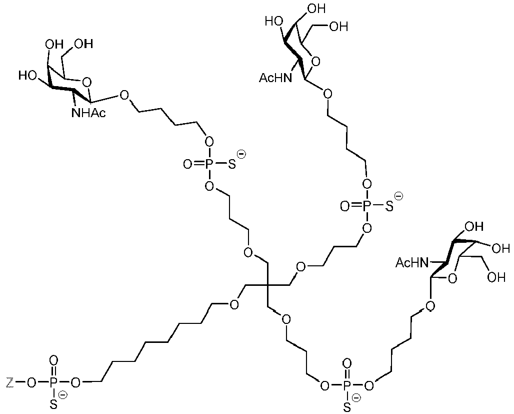

43. A nucleic acid of any preceding claim, wherein the ligand comprises:

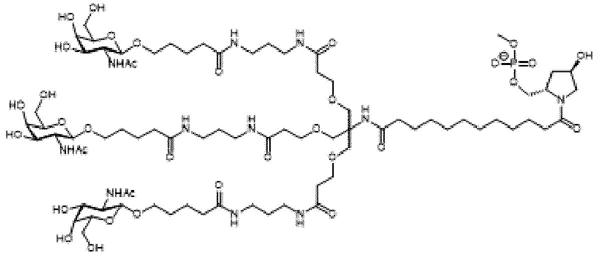

44. A composition comprising a nucleic acid as defined in any preceding claim and a formulation comprising:
i) a cationic lipid, or a pharmaceutically acceptable salt thereof;
ii) a steroid;
iii) a phosphatidylethanolamine phospholipid;
iv) a PEGylated lipid.

45. A composition according to claim 44, wherein in the formulation the content of the cationic lipid component is from about 55 mol% to about 65 mol% of the overall lipid content of the lipid formulation, preferably about 59 mol% of the overall lipid content of the lipid formulation.

46. A composition as claimed in claim 44, wherein the formulation comprises;
A cationic lipid having the structure; 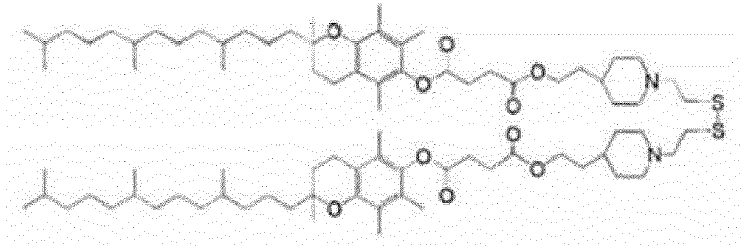 the steroid has the structure; 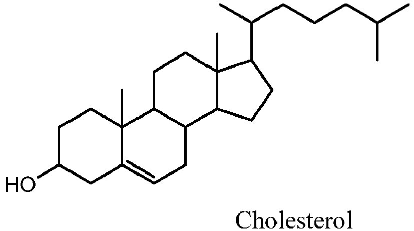 the phosphatidylethanolamine phospholipid has the structure; 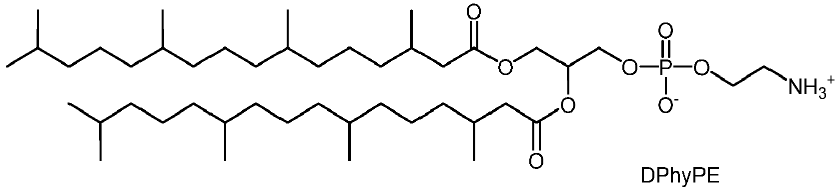 and the PEGylated lipid has the structure;

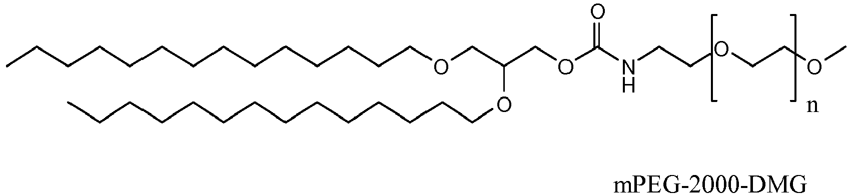

47. A composition comprising a nucleic acid of any preceding claim and a physiologically acceptable excipient.

48. A nucleic acid according to any preceding claim for use in the treatment of a disease or disorder.

49. Use of a nucleic acid according to any preceding claim in the manufacture of a medicament for treating a disease or disorder.

50. A method of treating a disease or disorder comprising administration of a composition comprising a nucleic acid according to any one preceding claim to an individual in need of treatment.

51. The method of claim 50, wherein the nucleic acid molecule is administered to the subject subcutaneously or intravenously.

52. A process of making a nucleic acid of any of claims 1 to 51.


SEQUENCE LISTING

<110>  Silence Therapeutics GmbH

<120>  Inverted Nucleotides

<130>  P110518EP/AC

<160>  20

<170>  PatentIn version 3.5

<210>  1
<211>  19
<212>  RNA
<213>  Artificial Sequence

<220>
<223>  TMPJH01A

<400>  1
aaccagaaga agcagguga                                                    19

<210>  2
<211>  19
<212>  RNA
<213>  Artificial Sequence

<220>
<223>  TMPJH01B

<400>  2
ucaccugcuu cuucugguu                                                    19

<210>  3
<211>  19
<212>  RNA
<213>  Artificial Sequence

<220>
<223>  TMPJH40A

<400>  3
aaccagaaga agcagguga                                                    19

<210>  4
<211>  18
<212>  RNA
<213>  Artificial Sequence

<220>
<223>  TMPJH40B

<400>  4
ucaccugcuu uucugguu                                                     18

<210>  5
<211>  20
<212>  RNA

<213>  Artificial Sequence

<220>
<223>  TMPJH41A

<400>  5
aaccagaaga agcaggugaa                                                    20


<210>  6
<211>  20
<212>  RNA
<213>  Artificial Sequence

<220>
<223>  TMPJH41B

<400>  6
ucaccugcuu cuucugguua                                                    20


<210>  7
<211>  20
<212>  RNA
<213>  Artificial Sequence

<220>
<223>  TMPJH42A

<400>  7
aaccagaaga agcaggugau                                                    20


<210>  8
<211>  20
<212>  RNA
<213>  Artificial Sequence

<220>
<223>  TMPJH42B

<400>  8
ucaccugcuu cuucugguuu                                                    20


<210>  9
<211>  20
<212>  RNA
<213>  Artificial Sequence

<220>
<223>  TMPJH43A

<400>  9
aaccagaaga agcaggugac                                                    20


<210>  10
<211>  20
<212>  RNA
<213>  Artificial Sequence

<220>

<223>  TMPJH43B

<400>  10
ucaccugcuu cuucugguuc                                                   20


<210>  11
<211>  20
<212>  RNA
<213>  Artificial Sequence

<220>
<223>  TMPJH44A

<400>  11
aaccagaaga agcaggugag                                                   20


<210>  12
<211>  20
<212>  RNA
<213>  Artificial Sequence

<220>
<223>  TMPJH44B

<400>  12
ucaccugcuu cuucugguug                                                   20


<210>  13
<211>  20
<212>  RNA
<213>  Artificial Sequence

<220>
<223>  TMPJH45A

<400>  13
aaccagaaga agcaggugaa                                                   20


<210>  14
<211>  20
<212>  RNA
<213>  Artificial Sequence

<220>
<223>  TMPJH45B

<400>  14
ucaccugcuu cuucugguua                                                   20


<210>  15
<211>  20
<212>  RNA
<213>  Artificial Sequence

<220>
<223>  TMPJH46A

<400>  15

aaccagaaga agcaggugau                                                    20

<210>  16
<211>  20
<212>  RNA
<213>  Artificial Sequence

<220>
<223>  TMPJH46B

<400>  16
ucaccugcuu cuucugguuu                                                    20

<210>  17
<211>  20
<212>  RNA
<213>  Artificial Sequence

<220>
<223>  TMPJH47A

<400>  17
aaccagaaga agcaggugac                                                    20

<210>  18
<211>  20
<212>  RNA
<213>  Artificial Sequence

<220>
<223>  TMPJH47B

<400>  18
ucaccugcuu cuucugguuc                                                    20

<210>  19
<211>  20
<212>  RNA
<213>  Artificial Sequence

<220>
<223>  TMPJH48A

<400>  19
aaccagaaga agcaggugag                                                    20

<210>  20
<211>  20
<212>  RNA
<213>  Artificial Sequence

<220>
<223>  TMPJH48B

<400>  20
ucaccugcuu cuucugguug                                                    20